(19) Europäisches Patentamt
European Patent Office
Office européen des brevets

(11) **EP 1 673 472 B1**

(12) **EUROPEAN PATENT SPECIFICATION**

(45) Date of publication and mention
of the grant of the patent:
**09.02.2011 Bulletin 2011/06**

(21) Application number: **04765830.7**

(22) Date of filing: **05.10.2004**

(51) Int Cl.:
***C12Q 1/68*** (2006.01)

(86) International application number:
**PCT/EP2004/011123**

(87) International publication number:
**WO 2005/040415 (06.05.2005 Gazette 2005/18)**

(54) **USE OF GENETIC POLYMORPHISMS TO PREDICT DRUG-INDUCED HEPATOTOXICITY**

VERWENDUNG GENETISCHER POLYMORPHISMEN ZUR VORHERSAGE ARZNEISTOFFINDUZIERTER HEPATOTOXIZITÄT

UTILISATION DE POLYMORPHISMES GENETIQUES POUR LA PREDICTION D'HEPATOTOXICITE INDUITE PAR MEDICAMENT

(84) Designated Contracting States:
**AT BE BG CH CY CZ DE DK EE ES FI FR GB GR HU IE IT LI LU MC NL PL PT RO SE SI SK TR**

(30) Priority: **06.10.2003 US 508972 P**

(43) Date of publication of application:
**28.06.2006 Bulletin 2006/26**

(73) Proprietors:
• **Novartis AG**
**4056 Basel (CH)**
Designated Contracting States:
**BE BG CH CY CZ DE DK EE ES FI FR GB GR HU IE IT LI LU MC NL PL PT RO SE SI SK TR**
• **Novartis Pharma GmbH**
**1230 Wien (AT)**
Designated Contracting States:
**AT**

(72) Inventors:
• **McCULLOUGH, Karen**
**Olney, MD 20832 (US)**
• **WOLFGANG, Curt, Douglas**
**Germantown, MD 20874 (US)**

(74) Representative: **Leon, Susanna Iris et al**
**Novartis AG**
**Corporate Intellectual Property**
**P.O. Box**
**4002 Basel (CH)**

(56) References cited:
**EP-A- 1 334 971**       **WO-A-03/102237**
**US-A1- 2001 034 032**

• GENC M R ET AL: "Polymorphism in intron 2 of the interleukin-1 receptor antagonist gene, local midtrimester cytokine response to vaginal flora, and subsequent preterm birth" AMERICAN JOURNAL OF OBSTETRICS & GYNECOLOGY, MOSBY, ST LOUIS, MO, US, vol. 191, no. 4, October 2004 (2004-10), pages 1324-1330, XP004621293 ISSN: 0002-9378
• MCGARRY F ET AL: "A polymorphism within the interleukin 1 receptor antagonist (IL-1Ra) gene is associated with ankylosing spondylitis" RHEUMATOLOGY, OXFORD UNIVERSITY PRESS, LONDON, GB, vol. 40, no. 12, December 2001 (2001-12), pages 1359-1364, XP002290518 ISSN: 1462-0324
• KATILA H ET AL: "POLYMORPHISMS OF THE INTERLEUKIN-1 GENE COMPLEX IN SCHIZOPHRENIA" MOLECULAR PSYCHIATRY, BASINGSTOKE, GB, vol. 4, no. 2, March 1999 (1999-03), pages 179-181, XP009033565 ISSN: 1359-4184

**Description**

FIELD OF THE INVENTION

This invention relates generally to the analytical testing of tissue samples *in vitro,* and more particularly to the analysis of genetic polymorphisms as biomarkers for predicting the occurrence of drug-induced hepatotoxicity.

DESCRIPTION OF THE RELATED ART

Among the disorders that arise from dysfunction of the microvasculature of diabetic patients is retinopathy, which manifests itself clinically as vision impairment and can result in blindness. Diabetic retinopathy is characterized by microaneurysms, excessive vascular permeability, areas of retinal nonperfusion, and retinal neovascularization. Much evidence suggests a causal link between high blood glucose levels and the development of the underlying lesions responsible for deficits in organ function. For a review, see Way KJ et al., Diabetic Medicine 18: 945-59 (2001).

Among the effects of hyperglycemia is the over-activation of the diacylglycerol (DAG) - protein kinase C (PKC) signal transduction pathway. Both cell culture experiments and animal models of diabetes demonstrate excessive levels and activity of DAG and PKC in vascular endothelial cells. Koya D & King GL, Diabetes 47: 859-866 (1998); Ishii H et al., J Mol Med 76: 21-31 (1998) and Way KJ et al., Trends Pharmacol Sci 21: 181-7 (2000). Activation of many of the isoforms of PKC serine-threonine kinases is dependent on DAG, a cleavage product of membrane phospholipids. Among the activated isoforms of PKC serine-threonine kinases is the predominant isoform PKCβ, which is associated with diabetic retinopathy. DAG is usually generated by agonist-stimulated hydrolysis of membrane phospholipids, but also can be synthesized *de novo* by direct metabolism of glucose. Dunlop ME & Larkins RG, Biochem Biophys Res Commun 132: 467-73 (1985); Ishii H et al., J Mol Med 76: 21-31 (1998). In response to hyperglycemia, *de novo* synthesis of DAG increases substantially, resulting in the activation of PKCβ. Ishii H et al., J Mol Med 76: 21-31 (1998).

As a consequence of continual activation of the DAG-PKC pathway, many aspects of vascular function are affected. Cytokine activation and leukocyte adhesion are stimulated; blood flow and microvessel contractility are altered; and extracellular matrix synthesis increases, resulting in the thickening of basement membranes. The retinal microenvironment becomes ischemic as a result of the aforementioned changes. Expression of vascular endothelial growth factor (VEGF), a potent stimulator of neovascularization, is upregulated in response to ischemia and by other PKCβ-dependent mechanisms. Aiello LP et al., Diabetes 46: 1473-80 (1997).

N-benzoyl-staurosporine (PKC412) is an inhibitor of both PKC and an essential VEGF receptor, KDR (Kinase insert Domain-containing Receptor, also known as VEGF-R2). N-benzoyl-staurosporine is being developed for several indications, including the treatment of diabetic macular edema. See, U.S. Pat. No. 6,214,819. See also, U.S. Pat. Appln. 20030119812, 20030125343 and 20030153551. Although a promising medication, treatment with N-benzoyl-staurosporine can result in known side effects, including liver toxicity. Thus, there is a need in the art for reducing the side effects of side effects of N-benzoyl-staurosporine.

SUMMARY OF THE INVENTION

The invention provides methods for determining subjects who are at risk for developing drug-induced hepatotoxicity. In one embodiment, the invention provides for the use of genomic analysis to identify patients at risk for experiencing hepatotoxicity during staurosporine therapy. In a particular embodiment, the staurosporine therapy involves the administration of N-benzoyl-staurosporine for treating diabetic macular edema. The hepatotoxicity prediction involves the determination of serum aspartate transaminase (AST) levels. In another embodiment, the invention provides methods for determining optimal treatment strategies for these patients.

The disclosure also provides clinical assays, kits and reagents for predicting hepatotoxicity prior to taking a drug. In one embodiment, the kits contain reagents for determining genetic polymorphisms in the *IL1A* gene. In a particular embodiment, the genetic polymorphism is at the PG locus ID 279 of the *IL1A* gene. In assays of genetic polymorphism of PG locus ID 279, the CC genotype (SEQ ID NO:1) is a biomarker for predictions of higher risk of hepatotoxicity, while the CT genotype (SEQ ID NOS:1 and 2) and TT genotype (SEQ ID NO:2) are biomarkers for a lower risk of hepatotoxicity. In another embodiment, the kits contain reagents for determining genetic polymorphisms in the *IL1A* gene. In a particular embodiment, the genetic polymorphism is at the PG locus ID 302 of the *IL1A* gene. In assays of genetic polymorphism of PG locus ID 302, the GG genotype (SEQ ID NO:3) is a biomarker for predictions of higher risk of hepatotoxicity, while the GT genotype (SEQ ID NOS:3 and 4) and TT genotype (SEQ ID NO:4) are biomarkers for a lower risk of hepatotoxicity.

BRIEF DESCRIPTION OF THE DRAWINGS

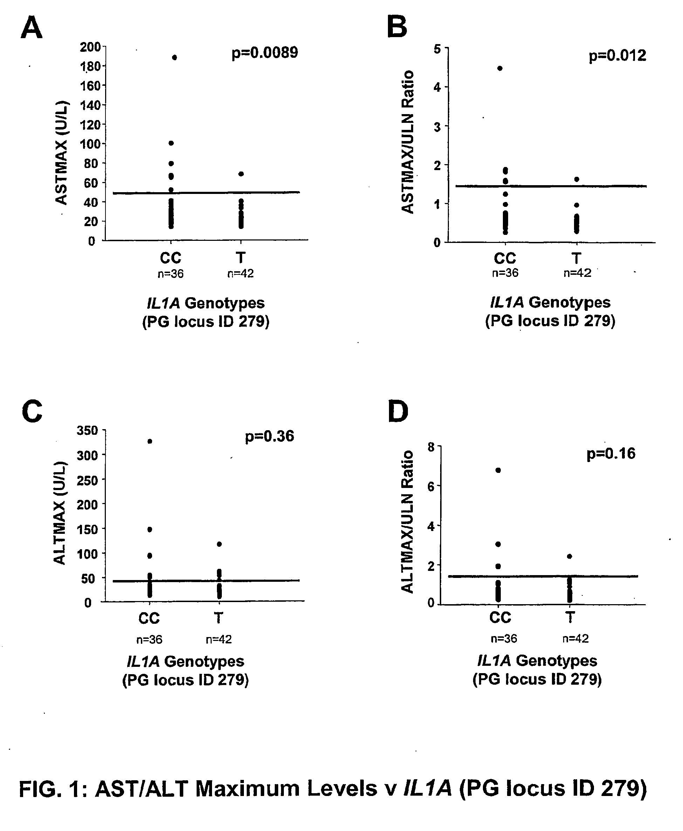

FIG. 1 shows the AST/ALT (aspartate aminotransferase / alanine aminotransferase) maximum levels vs. *IL1A*

(interleukin 1-alpha; PG locus ID 279). The scatter plots show (A) maximum aspartate aminotransferase levels, (B) ratio of ASTMAX and the upper limit of normal (ULN), (C) maximum alanine aminotransferase levels and (D) ratio of ALT MAX and ULN for subjects in the clinical trial with genotypes of CC or T (CT or TT) for IL1A PG locus ID 279. The upper limit of normal for aspartate aminotransferase is 42 U/L for ages 3-64 and 55 U/L for 65 and over and for alanine aminotransferase is 48 U/L for all ages. ULNs are indicated by a line.

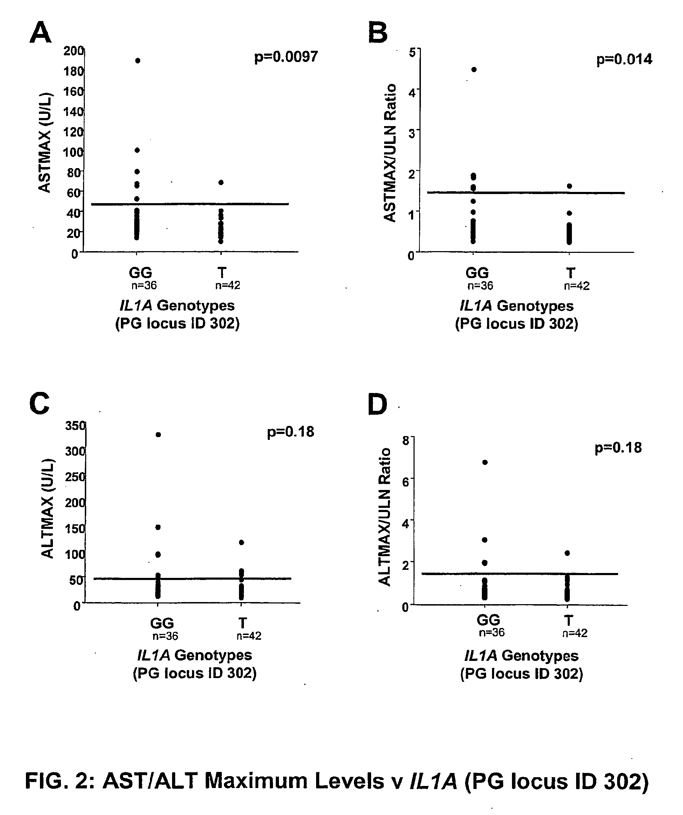

FIG. 2 shows the AST/ALT (aspartate aminotransferase / alanine aminotransferase) maximum levels vs. IL1A (interleukin 1-alpha; PG locus ID 302). The scatter plots show plots of (A) maximum alanine aminotransferase levels, (B) ratio of ASTMAX and ULN, (C) maximum ALT levels and (D) ratio of ALT MAX and ULN for subjects in the clinical trial with genotypes of G or T (GT or TT) for IL1A PG locus ID 302. The upper limit of normal for aspartate aminotransferase is 42 U/L for ages 3-64 and 55 U/L for 65 and over and for alanine aminotransferase is 48 U/L for all ages. ULNs are indicated by a line.

## DETAILED DESCRIPTION OF THE INVENTION

The invention advantageously provides a way to determine whether a patient will experience hepatotoxicity during drug treatment, prior to actually taking the drugs. The disclosure thus provides safer treatment regimens for patients by helping clinicians to either (1) alter the dose of the drug, (2) provide additional or alternative concomitant medication or (3) choose not to prescribe that drug for that patient.

Relevant genetic polymorphisms were identified in a multicenter, randomized, double-masked placebo-controlled dose finding phase II trial of N-benzoyl-staurosporine, which was conducted in subjects with diabetic macular edema. The safety of N-benzoyl-staurosporine was evaluated in the subjects and additional pharmacokinetic information on was collected. While N-benzoyl-staurosporine showed a good safety profile, nine of the 140 subjects that were enrolled in the clinical trial experienced hepatotoxicity, as defined by fold increases in liver transaminases over the upper limit of normal (ULN). Subjects were flagged as having experienced hepatotoxicity if either aspartate aminotransferase (AST) or alanine aminotransferase (ALT) had fold elevations over the upper limit of normal on visit 3, 4 or 5.

Of the eighteen single nucleotide polymorphisms (SNP) from seven genes that were genotyped, two in the *inter-leukin 1-alpha* (*IL1A*) gene were associated with the maximum serum aspartate transaminase level recorded on visits 3, 4 or 5. *IL1A* encodes an inflammatory cytokine that plays a pivotal role in mediating acute phase responses. One *IL1A* polymorphism is located in the promoter region of *IL1A* and the other results in a serine to alanine substitution at amino acid position 114. These results suggest that polymorphisms in *IL1A* or a gene located near it on 2q14, may be directly involved with the onset of liver toxicity following, administration of N-benzoyl-staurosporine.

As used herein, a polymorphism in the *IL1A* genetic locus is "predictive" of a "high" risk of hepatotoxicity when genetic polymorphism correlates significantly with the development of drug-induced hepatotoxicity or with elevated levels of serum aspartate transaminase. See, for example, below, where the CC genotype at the PG locus ID 279 and the GG genotype at the PG locus ID 302 are predictive of a high risk of hepatotoxicity. As used herein, a polymorphism in the *IL1A* genetic locus is "predictive" of a "low" risk of hepatotoxicity when genetic polymorphism correlate significantly with the lack of development of hepatotoxicity. See, for example, below, where the CT or TT genotype at the PG locus ID 279 and the GT or TT genotype at the PG locus ID 302 are predictive of a low risk of hepatotoxicity. Determinations of significance (p values) can be determined by analysis of variance (ANOVA) or Fisher's Exact tests. Determinations of one SNP polymorphism at a certain IL1A genetic site as having a high risk for developing hepatotoxicity and another SNP polymorphism at that IL1A genetic site as having a low risk for developing hepatotoxicity can be combined for greater accuracy of determination. For PG locus IDs 279 and 302, associations between *IL1A* polymorphisms and serum aspartate transaminase levels had p values of 0.0089 and 0.0097.

These results can reasonably be extrapolated to the prediction of hepatotoxicity in patients following the administration of any staurosporine derivatives, based upon the structural similarity and modes of action in the liver of staurosporine derivatives. Among the staurosporine derivatives are those described in U.S. Pat. No. 5,093,330. Preferred compounds are N-acylstaurosporines and their pharmaceutically acceptable salts, including N-(2-aminoacetyl)staurosporine; N-(3,5-dinitrobenzoyl)-staurosporine; N-(3-carboxypropionyl)staurosporine; N-(3-fluorobenzoyl)-staurosporine; N-(3-nitrobenzoyl)staurosporine; N-(4-carboxybenzoyl)staurosporine; N-[(tert-butoxycarbonylamino)-acetyl]-staurosporine; N-alanylstaurosporine; N-benzoyl staurosporine; N-carboxymethyl-staurosporine; N-ethyl-staurosporine; N-methylaminothiocarbonylstaurosporine; N-phenylcarbamoylstaurosporine; N-tert-butoxycarbonylstaurosporine; and N-trifluoracetylstaurosporine.

Moreover, the results can be extrapolated to the prediction of hepatotoxicity in patients who are being treated for diseases other than diabetic macular edema. The method of the invention is applicable to vertebrate subjects, particularly to mammalian subjects, more particularly to human subjects. The invention is particularly applicable to diabetic subjects.

The diagnosis of hepatotoxicity can be accomplished using assays of serum enzyme levels. Serum enzyme assays indicative of liver dysfunction are well-known to those of skill in the medical arts and routine in hospital laboratories.

For a definition of hepatotoxicity based upon serum levels of aspartate transaminase (AST) and used in the EXAMPLE: The definition of hepatotoxicity used in this analysis was based on fold increases in serum aspartate transaminase or alanine aminotransferase over the upper limit of normal (ULN) on visits 3, 4 or 5. The upper limit of normal for serum aspartate transaminase is 42 U/L for ages 3-64 and 55 U/L for ages 65 and up; for serum alanine aminotransferase the upper limit of normal is 48 U/L (Smithkline Beecham Clinical Laboratories Reference Alert Ranges). While an elevation of either enzyme at visit 3, 4 or 5 constituted hepatotoxicity, transaminase elevations were disregarded during subsequent visits when the drug was not being administered. Furthermore, subjects who had elevated liver function tests at baseline (visit 2) were not flagged as having experienced hepatotoxicity, regardless of the elevation in their enzyme levels following administration of the drug. Nine subjects were flagged as having experienced hepatotoxicity. Of these, six consented to clinical pharmacogenetic analysis.

Individuals carrying polymorphic alleles may be detected at the DNA, the RNA, or the protein level using a variety of techniques that are well known in the art. Strategies for identification and detection are described in *e.g.* EP 730,663, EP 717,113, and PCT US97/02102. The methods of the invention may involve the detection of pre-characterized polymorphisms. That is, the genotyping location and nature of polymorphic forms present at a site have already been determined (see, discussion above regarding interrogated genes). The availability of this information allows sets of probes to be designed for specific identification of the known polymorphic forms. The identification of alleles containing single nucleotide polymorphisms may involve the amplification of DNA from target samples. This can be accomplished by *e.g.,* PCR. See generally PCR Technology: Principles and Applications for DNA Amplification, (ed. Erlich, Freeman Press, New York, New York, 1992); PCR Protocols: A Guide to Methods and Application (eds. Innis, et al., Academic Press, San Diego, Calif., 1990). The detection of polymorphisms in specific DNA sequences, can be accomplished by a variety of methods including, but not limited to, restriction-fragment-length-polymorphism detection based on allele-specific restriction-endonuclease cleavage (Kan & Dozy, Lancet II:910-912 (1978)), hybridization with allele-specific oligonucleotide probes (Wallace et al, Nucl. Acids Res. 6:3543-3557 (1978)), including immobilized oligonucleotides (Saiki et al., Proc. Natl. Acad. Sci. USA, 86:6230-6234 (1969)) or oligonucleotide arrays (Maskos & Southern, Nucl. Acids Res. 21:2269-2270 (1993)), allele-specific PCR (Newton et al., Nucl. Acids Res. 17:2503-2516 (1989)), mismatch-repair detection (MRD) (Faham & Cox, Genome Res. 5:474-482 (1995)), binding of MutS protein (Wagner et al., Nucl. Acids Res. 23:3944-3948 (1995), denaturing-gradient gel electrophoresis (DGGE) (Fisher & Lerman, Proc. Natl. Acad. Sci. U.S.A. 80:1579-1583 (1983)), single-strand-conformation-polymorphism detection (Orita et al., Genomics 5:874-879 (1983)), RNAse cleavage at mismatched base-pairs (Myers et al., Science 230:1242 (1985)), chemical (Cotton et al., Proc. Natl. Acad. Sci. U.S.A., 8Z:4397-4401 (1988)) or enzymatic (Youil et al., Proc. Natl. Acad. Sci. U.S.A. 92:87-91 (1995)) cleavage of heteroduplex DNA, methods based on allele specific primer extension (Syvanen et al., Genomics 8:684-692 (1990)), genetic bit analysis (GBA) (Nikiforov et al., Nucl. Acids Res. 22:4167-4175 (1994)), the oligonucleotide-ligation assay (OLA) (Landegren et al., Science 241:1077 (1988)), the allele-specific ligation chain reaction (LCR) (Barrany, Proc. Natl. Acad. Sci. U.S.A. 88:189-193 (1991)), gap-LCR (Abravaya et al., Nucl. Acids Res. 23:675-682 (1995)), radioactive and/or fluorescent DNA sequencing using standard procedures well known in the art, and peptide nucleic acid (PNA) assays (Orum et al., Nucl. Acids Res. 21:5332-5356 (1993); Thiede et al., Nucl. Acids Res. 24:983-984 (1996)). Additional guidance is provided by Sambrook J et al., Molecular Cloning: A Laboratory Manual, Third Edition (Cold Spring Harbor Press, Cold Spring Harbor, New York, 2000).

Guidance for the use of N-benzoyl-staurosporine and related staurospaurine derivatives is provided in U.S. Pat. Nos. 5,744,460; 5,827,846; 6,018,042; 6,153,599 and 6,214,819. Additional guidance for the use of N-benzoyl-staurosporine related staurospaurine derivatives for treating ocular neovascular diseases and in decreasing capillary permeability in the retina is provided in U.S. Pat. Applns. 20030119812, 20030125343 and 20030153551

*Single Nucleotide Polymorphisms.* Sequence variation in the human genome consists primarily of single nucleotide polymorphisms ("SNPs") with the remainder of the sequence variations being short tandem repeats (including micro-satellites), long tandem repeats (mini-satellite) and other insertions and deletions. A SNP is a position at which two alternative bases occur at appreciable frequency (*i.e.* >1%) in the human population. A SNP is said to be "allelic" in that due to the existence of the polymorphism, some members of a species may have the unmutated sequence (i.e., the original "allele") whereas other members may have a mutated sequence (i.e., the variant or mutant allele). In the simplest case, only one mutated sequence may exist, and the polymorphism is said to be diallelic. The occurrence of alternative mutations can give rise to triallelic polymorphisms, *etc.* SNPs are widespread throughout the genome and SNPs that alter the function of a gene may be direct contributors to phenotypic variation. Due to their prevalence and widespread nature, SNPs have potential to be important tools for locating genes that are involved in human disease conditions, *see e.g.,* Wang et al., Science 280: 1077-1082 (1998), which discloses a pilot study in which 2,227 SNPs were mapped over a 2.3 megabase region of DNA.

An association between a single nucleotide polymorphisms and a particular phenotype does not indicate or require that the SNP is causative of the phenotype. Instead, such an association may indicate only that the SNP is located near the site on the genome where the determining factors for the phenotype exist and therefore is more likely to be found in association with these determining factors and thus with the phenotype of interest. Thus, a SNP may be in linkage disequilibrium (LD) with the 'true' functional variant. LD, also known as allelic association exists when alleles at two distinct locations of the genome are more highly associated than expected.

Thus a SNP may serve as a marker that has value by virtue of its proximity to a mutation that causes a particular phenotype.

SNPs that are associated with disease may also have a direct effect on the function of the gene in which they are located. A sequence variant may result in an amino acid change or may alter exon-intron splicing, thereby directly modifying the relevant protein, or it may exist in a regulatory region, altering the cycle of expression or the stability of the mRNA, see Nowotny P Current Opinions in Neurobiology 11:637-641 (2001).

It is increasingly clear that the risk of developing many common disorders and the metabolism of medications used to treat these conditions are substantially influenced by underlying genomic variations, although the effects of any one variant might be small.

Therefore, an association between a SNP and a clinical phenotype suggests, (1) the SNP is functionally responsible for the phenotype or, (2) there are other mutations near the location of the SNP on the genome that cause the phenotype. The 2nd possibility is based on the biology of inheritance. Large pieces of DNA are inherited and markers in close proximity to each other may not have been recombined in individuals that are unrelated for many generations, *i.e.*, the markers are in linkage disequlibrium (LD).

*Identification and characterization of SNPs.* Many different techniques can be used to identify and characterize SNPs, including single-strand conformation polymorphism analysis, heteroduplex analysis by denaturing high-performance liquid chromatography (DHPLC), direct DNA sequencing and computational methods, *see* Shi MM, Clin Chem 47: 164-172 (2001). Thanks to the wealth of sequence information in public databases, computational tools can be used to identify SNPs *in silico* by aligning independently submitted sequences for a given gene (either cDNA or genomic sequences). Comparison of SNPs obtained experimentally and by *in silico* methods showed that 55% of candidate SNPs found by SNPFinder(http://lpgws.nci.nih.gov:82/perl/snp/snp_cgi.pl) have also been discovered experimentally, *see,* Cox et al. Hum Mutat 17:141-150 (2001). However, these *in silico* methods could only find 27% of true SNPs.

The most common SNP typing methods currently include hybridization, primer extension and cleavage methods. Each of these methods must be connected to an appropriate detection system. Detection technologies include fluorescent polarization, (see Chan X et al. Genome Res 9:492-499 (1999)), luminometric detection of pyrophosphate release (pyrosequencing), (see Ahmadiian A et al., Anal Biochem 280:103-10 (2000)), fluorescence resonance energy transfer (FRET)-based cleavage assays, DHPLC, and mass spectrometry, (see Shi MM, Clin Chem 47:164-172 (2001) and U.S. Pat. No. 6,300,076 B1). Other methods of detecting and characterizing SNPs are those disclosed in U.S. Patents No. 6,297,018 B1 and 6,300,063 B1.

In a particularly preferred embodiment the detection of the polymorphism can be accomplished by means of so called INVADER™ technology (available from Third Wave Technologies Inc. Madison, Wisconsin, USA). In this assay, a specific upstream "invader" oligonucleotide and a partially overlapping downstream probe together form a specific structure when bound to complementary DNA template. This structure is recognized and cut at a specific site by the Cleavase enzyme, and this results in the release of the 5' flap of the probe oligonucleotide. This fragment then serves as the "invader" oligonucleotide with respect to synthetic secondary targets and secondary fluorescently labelled signal probes contained in the reaction mixture. This results in specific cleavage of the secondary signal probes by the Cleavase enzyme. Fluorescence signal is generated when this secondary probe, labelled with dye molecules capable of fluorescence resonance energy transfer, is cleaved. Cleavases have stringent requirements relative to the structure formed by the overlapping DNA sequences or flaps and can, therefore, be used to specifically detect single base pair mismatches immediately upstream of the cleavage site on the downstream DNA strand. See Ryan D et al. Molecular Diagnosis 4(2):135-144 (1999) and Lyamichev V et al. Nature Biotechnology 17:292-296 (1999), see also US Patents 5,846,717 and 6,001,567.

In some embodiments, a composition contains two or more differently labelled genotyping oligonucleotides for simultaneously probing the identity of nucleotides at two or more polymorphic sites. It is also contemplated that primer compositions may contain two or more sets of allele-specific primer pairs to allow simultaneous targeting and amplification of two or more regions containing a polymorphic site.

Genotyping oligonucleotides may also be immobilized on or synthesized on a solid surface such as a microchip, bead or glass slide (see, *e.g.*, WO 98/20020 and WO 98/20019). Such immobilized genotyping oligonucleotides may be used in a variety of polymorphism detection assays, including but not limited to probe hybridization and polymerase extension assays. Immobilized genotyping oligonucleotides may comprise an ordered array of oligonucleotides designed to rapidly screen a DNA sample for polymorphisms in multiple genes at the same time.

An allele-specific oligonucleotide primer has a 3' terminal nucleotide, or preferably a 3' penultimate nucleotide, that is complementary to only one nucleotide of a particular SNP, thereby acting as a primer for polymerase-mediated extension only if the allele containing that nucleotide is present. Allele-specific oligonucleotide primers hybridizing to either the coding or noncoding strand are also contemplated. An ASO primer for detecting gene polymorphisms could be developed using techniques known to those of skill in the art.

Other genotyping oligonucleotides hybridize to a target region located one to several nucleotides downstream of one of the novel polymorphic sites identified herein. Such oligonucleotides are useful in polymerase-mediated primer extension methods for detecting one of the novel polymorphisms described herein and therefore such genotyping oligonucleotides are referred to herein as "primer-extension oligonucleotides". In a preferred embodiment, the 3'-terminus of a primer-extension oligonucleotide is a deoxynucleotide complementary to the nucleotide located immediately adjacent to the polymorphic site.

In another embodiment, the disclosure provides a kit comprising at least two genotyping oligonucleotides packaged in separate containers. The kit may also contain other components such as hybridization buffer (where the oligonucleotides are to be used as a probe) packaged in a separate container. Alternatively, where the oligonucleotides are to be used to amplify a target region, the kit may contain, packaged in separate containers, a polymerase and a reaction buffer optimized for primer extension mediated by the polymerase, such as PCR.

The above described oligonucleotide compositions and kits are useful in methods for genotyping and/or haplotyping the gene in an individual. As used herein, the terms " genotype" and " haplotype" mean the genotype or haplotype containing the nucleotide pair or nucleotide, respectively, that is present at one or more of the novel polymorphic sites described herein and may optionally also include the nucleotide pair or nucleotide present at one or more additional polymorphic sites in the gene. The additional polymorphic sites may be currently known polymorphic sites or sites that are subsequently discovered.

One embodiment of the genotyping method involves isolating from the individual a nucleic acid mixture comprising the two copies of the gene, or a fragment thereof, that are present in the individual, and determining the identity of the nucleotide pair at one or more of the polymorphic sites in the two copies to assign a genotype to the individual. As will be readily understood by the skilled artisan, the two "copies" of a gene in an individual may be the same allele or may be different alleles. In a particularly preferred embodiment, the genotyping method comprises determining the identity of the nucleotide pair at each polymorphic site.

Typically, the nucleic acid mixture is isolated from a biological sample taken from the individual, such as a blood sample or tissue sample. Suitable tissue samples include whole blood, semen, saliva, tears, urine, fecal material, sweat, buccal smears, skin and hair. The nucleic acid mixture may be comprised of genomic DNA, mRNA, or cDNA and, in the latter two cases, the biological sample must be obtained from an organ in which the gene is expressed. Furthermore it will be understood by the skilled artisan that mRNA or cDNA preparations would not be used to detect polymorphisms located in introns or in 5' and 3' nontranscribed regions. If a gene fragment is isolated, it must contain the polymorphic site(s) to be genotyped.

One embodiment of the haplotyping method comprises isolating from the individual a nucleic acid molecule containing only one of the two copies of the gene, or a fragment thereof, that is present in the individual and determining in that copy the identity of the nucleotide at one or more of the polymorphic sites in that copy to assign a haplotype to the individual. The nucleic acid may be isolated using any method capable of separating the two copies of the gene or fragment, including but not limited to, one of the methods described above for preparing isogenes, with targeted *in vivo* cloning being the preferred approach. As will be readily appreciated by those skilled in the art, any individual clone will only provide haplotype information on one of the two gene copies present in an individual. If haplotype information is desired for the individual's other copy, additional clones will need to be examined. Typically, at least five clones should be examined to have more than a 90% probability of haplotyping both copies of the gene in an individual. In a particularly preferred embodiment, the nucleotide at each of polymorphic site is identified.

In a preferred embodiment, a haplotype pair is determined for an individual by identifying the phased sequence of nucleotides at one or more of the polymorphic sites in each copy of the gene that is present in the individual. In a particularly preferred embodiment, the haplotyping method comprises identifying the phased sequence of nucleotides at each polymorphic site in each copy of the gene. When haplotyping both copies of the gene, the identifying step is preferably performed with each copy of the gene being placed in separate containers. However, it is also envisioned that if the two copies are labeled with different tags, or are otherwise separately distinguishable or identifiable, it could be possible in some cases to perform the method in the same container. For example, if first and second copies of the gene are labelled with different first and second fluorescent dyes, respectively, and an allele-specific oligonucleotide labelled with yet a third different fluorescent dye is used to assay the polymorphic site(s), then detecting a combination of the first and third dyes would identify the polymorphism in the first gene copy while detecting a combination of the second and third dyes would identify the polymorphism in the second gene copy.

In both the genotyping and haplotyping methods, the identity of a nucleotide (or nucleotide pair) at a polymorphic site(s) may be determined by amplifying a target region(s) containing the polymorphic site(s) directly from one or both copies of the gene, or fragment thereof, and the sequence of the amplified region(s) determined by conventional methods. It will be readily appreciated by the skilled artisan that only one nucleotide will be detected at a polymorphic site in individuals who are homozygous at that site, while two different nucleotides will be detected if the individual is heterozygous for that site. The polymorphism may be identified directly, known as positive-type identification, or by inference, referred to as negative-type identification. For example, where a SNP is known to be guanine and cytosine in a reference population, a site may be positively determined to be either guanine or cytosine for ail individual homozygous at that site, or both guanine and cytosine, if the individual is heterozygous at that site. Alternatively, the site may be negatively determined to be not guanine (and thus cytosine/cytosine) or not cytosine (and thus guanine/guanine).

In addition, the identity of the allele(s) present at any of the novel polymorphic sites described herein may be indirectly determined by genotyping a polymorphic site not disclosed herein that is in linkage disequilibrium with the polymorphic site that is of interest. Two sites are said to be in linkage disequilibrium if the presence of a particular variant at one site enhances the predictability of another variant at the second site (*see,* Stevens JC, Mol Diag 4:309-317 (1999)). Polymorphic sites in linkage disequilibrium with the presently disclosed polymorphic sites may be located in regions of the gene or in other genomic regions not examined herein. Genotyping of a polymorphic site in linkage disequilibrium with the novel polymorphic sites described herein may be performed by, but is not limited to, any of the above-mentioned methods for detecting the identity of the allele at a polymorphic site.

The target region(s) may be amplified using any oligonucleotide-directed amplification method, including but not limited to polymerase chain reaction (PCR) (U.S. Pat. No. 4,965,188), ligase chain reaction (LCR) (Barany et al., Proc Natl Acad Sci USA 88:189-193 (1991); PCT patent application WO 90/01069), and oligonucleotide ligation assay (OLA) (Landegren et al., Science 241:1077-1080 (1988)). Oligonucleotides useful as primers or probes in such methods should specifically hybridize to a region of the nucleic acid that contains or is adjacent to the polymorphic site. Typically, the oligonucleotides are between 10 and 35 nucleotides in length and preferably, between 15 and 30 nucleotides in length. Most preferably, the oligonucleotides are 20 to 25 nucleotides long. The exact length of the oligonucleotide will depend on many factors that are routinely considered and practiced by the skilled artisan.

Other known nucleic acid amplification procedures may be used to amplify the target region including transcription-based amplification systems (U.S. Pat. No. 5,130,238; EP 329,822; U.S. Pat. No. 5,169,766, WO 89/06700) and isothermal methods (Walker et al., Proc Natl Acad Sci USA 89:392-396 (1992)).

A polymorphism in the target region may also be assayed before or after amplification using one of several hybridization-based methods known in the art. Typically, allele-specific oligonucleotides are utilized in performing such methods. The allele-specific oligonucleotides may be used as differently labelled probe pairs, with one member of the pair showing a perfect match to one variant of a target sequence and the other member showing a perfect match to a different variant. In some embodiments, more than one polymorphic site may be detected at once using a set of allele-specific oligonucleotides or oligonucleotide pairs. Preferably, the members of the set have melting temperatures within 5°C and more preferably within 2°C, of each other when hybridizing to each of the polymorphic sites being detected.

Hybridization of an allele-specific oligonucleotide to a target polynucleotide may be performed with both entities in solution or such hybridization may be performed when either the oligonucleotide or the target polynucleotide is covalently or noncovalently affixed to a solid support. Attachment may be mediated, for example, by antibody-antigen interactions, poly-L-Lys, streptavidin or avidin-biotin, salt bridges, hydrophobic interactions, chemical linkages, UV cross-linking baking, etc. Allele-specific oligonucleotides may be synthesized directly on the solid support or attached to the solid support subsequent to synthesis. Solid-supports suitable for use in detection methods of the invention include substrates made of silicon, glass, plastic, paper and the like, which may be formed, for example, into wells (as in 96-well plates), slides, sheets, membranes, fibers, chips, dishes, and beads. The solid support may be treated, coated or derivatized to facilitate the immobilization of the allele-specific oligonucleotide or target nucleic acid.

The genotype or haplotype for the gene of an individual may also be determined by hybridization of a nucleic sample containing one or both copies of the gene to nucleic acid arrays and subarrays such as described in WO 95/11995. The arrays would contain a battery of allele-specific oligonucleotides representing each of the polymorphic sites to be included in the genotype or haplotype.

The identity of polymorphisms may also be determined using a mismatch detection technique, including but not limited to the RNase protection method using riboprobes (Winter et al., Proc Natl Acad Sci USA 82:7575 (1985); Meyers et al., Science 230:1242 (1985)) and proteins which recognize nucleotide mismatches, such as the *E. coli* mutS protein (Modrich P. Ann Rev Genet 25:229-253 (1991)). Alternatively, variant alleles can be identified by single strand conformation polymorphism (SSCP) analysis (Orita et al., Genomics 5:874-879 (1989); Humphries et al., in Molecular Diagnosis of Genetic Diseases, R. Elles, ed., pp. 321-340 (1996)) or denaturing gradient gel electrophoresis (DGGE) (Wartell et al., Nucl Acids Res 18:2699-2706 (1990); Sheffield et al., Proc Natl Acad Sci USA 86:232-236 (1989)).

A polymerase-mediated primer extension method may also be used to identify the polymorphism(s). Several such methods have been described in the patent and scientific literature and include the "Genetic Bit Analysis" method (WO 92/15712) and the ligase / polymerase mediated genetic bit analysis (U.S. Pat. No. 5,679,524). Related methods are disclosed in WO 91/02087, WO 90/09455, WO 95/17676, U.S. Pat. Nos. 5,302,509 and 5,945,283. Extended primers containing a polymorphism may be detected by mass spectrometry as described in U.S. Pat. No. 5,605,798. Another primer extension method is allele-specific PCR (Ruafio et al., Nucl Acids Res 17:8392 (1989); Ruafio et al., Nucl Acids Res 19:6877-6882 (1991); WO 93/22456; Turki et al., J Clin Invest 95:1635-1641 (1995)). In addition, multiple polymorphic sites may be investigated by simultaneously amplifying multiple regions of the nucleic acid using sets of allele-specific primers as described in WO 89/10414.

In a preferred embodiment, the haplotype frequency data for each ethnogeographic group is examined to determine whether it is consistent with Hardy-Weinberg equilibrium. Hardy-Weinberg equilibrium (D.L. Hartl et al., Principles of Population Genomics, 3rd Ed. (Sinauer Associates, Sunderland, MA, 1997) postulates that the frequency of finding the haplotype pair $H_1/H_2$ is equal to $P_{H-W}$ $(H_1/H_2) = 2p(H_1)$ $p$ $(H_2)$ if $H_1 \neq H_2$ and $P_{H-W}$ $(H_1/H_2) = p$ $(H_1)$ $p$ $(H_2)$ if $H_1 = H_2$. A statistically significant difference between the observed and expected haplotype frequencies could be due to one or more factors including significant inbreeding in the population group, strong selective pressure on the gene, sampling bias, and/or errors in the genotyping process. If large deviations from Hardy-Weinberg equilibrium are observed in an ethnogeographic group, the number of individuals in that group can be increased to see if the deviation is due to a sampling bias. If a larger sample size does not reduce the difference between observed and expected haplotype pair frequencies, then one may wish to consider haplotyping the individual using a direct haplotyping method such as, for example, CLASPER System™ technology (U.S. Pat. No. 5,866,404), SMD, or allele-specific long-range PCR (Michalotos-Beloin et al., Nucl Acids Res 24:4841-4843 (1996)).

In one embodiment of this method for predicting a haplotype pair, the assigning step involves performing the following analysis. First, each of the possible haplotype pairs is compared to the haplotype pairs in the reference population. Generally, only one of the haplotype pairs in the reference population matches a possible haplotype pair and that pair is assigned to the individual. Occasionally, only one haplotype represented in the reference haplotype pairs is consistent with a possible haplotype pair for an individual, and in such cases the individual is assigned a haplotype pair containing this known haplotype and a new haplotype derived by subtracting the known haplotype from the possible haplotype pair. In rare cases, either no haplotypes in the reference population are consistent with the possible haplotype pairs, or alternatively, multiple reference haplotype pairs are consistent with the possible haplotype pairs. In such cases, the individual is preferably haplotyped using a direct molecular haplotyping method such as, for example, CLASPER System™ technology (U.S. Pat. No. 5,866,404), SMD, or allele-specific long-range PCR (Michalotos-Beloin et al., Nucl Acids Res 24:4841-4843 (1996)).

The disclosure also provides a method for determining the frequency of a genotype or haplotype in a population. The method comprises determining the genotype or the haplotype pair for the gene that is present in each member of the population, wherein the genotype or haplotype comprises the nucleotide pair or nucleotide detected at one or more of the polymorphic sites in the gene, and calculating the frequency any particular genotype or haplotype is found in the population. The population may be a reference population, a family population, a same sex population, a population group, a trait population (*e.g.*, a group of individuals exhibiting a trait of interest such as a medical condition or response to a therapeutic treatment).

In another aspect of the disclosure, frequency data for genotypes and/or haplotypes found in a reference population are used in a method for identifying an association between a trait and a genotype or a haplotype. The trait may be any detectable phenotype, including but not limited to susceptibility to a disease or response to a treatment. The method involves obtaining data on the frequency of the genotype(s) or haplotype(s) of interest in a reference population as well as in a population exhibiting the trait. Frequency data for one or both of the reference and trait populations may be obtained by genotyping or haplotyping each individual in the populations using one of the methods described above. The haplotypes for the trait population may be determined directly or, alternatively, by the predictive genotype to haplotype approach described above.

In another embodiment, the frequency data for the reference and/or trait populations is obtained by accessing previously determined frequency data, which may be in written or electronic form. For example, the frequency data may be present in a database that is accessible by a computer. Once the frequency data is obtained, the frequencies of the genotype(s) or haplotype(s) of interest in the reference and trait populations are compared. In a preferred embodiment, the frequencies of all genotypes and/or haplotypes observed in the populations are compared. If a particular genotype or haplotype for the gene is more frequent in the trait population than in the reference population at a statistically significant amount, then the trait is predicted to be associated with that genotype or haplotype.

In a preferred embodiment statistical analysis is performed by the use of standard ANOVA tests with a Bonferoni correction and/or a bootstrapping method that simulates the genotype phenotype correlation many times and calculates a significance value. When many polymorphisms are being analyzed a correction to factor may be performed to correct for a significant association that might be found by chance. For statistical methods for use in the methods of this dislosure, see: Statistical Methods in Biology, 3rd edition, Bailey NTJ, (Cambridge Univ. Press, 1997); Introduction to Computational Biology, Waterman MS (CRC Press, 2000) and Bioinformatics, Baxevanis AD & Ouellette BFF editors (John Wiley & Sons, Inc., 2001).

In a preferred embodiment of the method, the trait of interest is a clinical response exhibited by a patient to some therapeutic treatment, for example, response to a drug targeting or response to a therapeutic treatment for a medical condition.

In another embodiment, a detectable genotype or haplotype that is in linkage disequilibrium with the genotype or haplotype of interest may be used as a surrogate marker. A genotype that is in linkage disequilibrium with a genotype may be discovered by determining if a particular genotype or haplotype for the gene is more frequent in the population that also demonstrates the potential surrogate marker genotype than in the reference population at a statistically significant amount, then the marker genotype is predicted to be associated with that genotype or haplotype and then can be used as a surrogate marker in place of the genotype.

*Definitions.* As used herein, "medical condition" includes but is not limited to any condition or disease manifested as one or more physical and/or psychological symptoms for which treatment is desirable, and includes previously and newly identified diseases and other disorders.

As used herein, the term "clinical response" means any or all of the following: a quantitative measure of the response, no response, and adverse response (i.e., side effects).

In order to deduce a correlation between clinical response to a treatment and a genotype or haplotype, data is obtained on the clinical responses exhibited by a population of individuals who received the treatment, hereinafter the "clinical population". This clinical data may be obtained by analyzing the results of a clinical trial that has already been run and/or the clinical data may be obtained by designing and carrying out one or more new clinical trials.

As used herein, the term "clinical trial" means any research study designed to collect clinical data on responses to a particular treatment, and includes but is not limited to phase I, phase II and phase III clinical trials. Standard methods are used to define the patient population and to enroll subjects.

It is preferred that the individuals included in the clinical population have been graded for the existence of the medical condition of interest. This grading of potential patients could employ a standard physical exam or one or more lab tests. Alternatively, grading of patients could use haplotyping for situations where there is a strong correlation between haplotype pair and disease susceptibility or severity.

The therapeutic treatment of interest is administered to each individual in the trial population and each individual's response to the treatment is measured using one or more predetermined criteria. It is contemplated that in many cases, the trial population will exhibit a range of responses and that the investigator will choose the number of responder groups (*e.g.*, low, medium, high) made up by the various responses. In addition, the gene for each individual in the trial population is genotyped and/or haplotyped, which may be done before or after administering the treatment.

After both the clinical and polymorphism data have been obtained, correlations between individual response and genotype or haplotype content are created. Correlations may be produced in several ways. In one method, individuals are grouped by their genotype or haplotype (or haplotype pair) (also referred to as a polymorphism group), and then the averages and standard deviations of clinical responses exhibited by the members of each polymorphism group are calculated.

These results are then analyzed to determine if any observed variation in clinical response between polymorphism groups is statistically significant. Statistical analysis methods which may be used are described in L.D. Fisher & G. vanBelle, Biostatistics: A Methodology for the Health Sciences (Wiley-Interscience, New York, 1993). This analysis may also include a regression calculation of which polymorphic sites in the gene give the most significant contribution to the differences in phenotype.

A second method for finding correlations between haplotype content and clinical responses uses predictive models based on error-minimizing optimization algorithms. One of many possible optimization algorithms is a genetic algorithm (R. Judson, "Genetic Algorithms and Their Uses in Chemistry" in Reviews in Computational Chemistry, Vol. 10, pp. 1- 73, K.B. Lipkowitz & D.B. Boyd, eds. (VCH Publishers, New York, 1997). Simulated annealing (Press et al., "Numerical Recipes in C: The Art of Scientific Computing", Cambridge University Press (Cambridge) 1992, Ch. 10), neural networks (E. Rich and K. Knight, "Artificial Intelligence", 2nd Edition (McGraw-Hill, New York, 1991, Ch. 18), standard gradient descent methods (Press *et al., supra* Ch. 10), or other global or local optimization approaches (see discussion in Judson, supra) could also be used.

Correlations may also be analyzed using analysis of variation (ANOVA) techniques to determine how much of the variation in the clinical data is explained by different subsets of the polymorphic sites in the gene. ANOVA is used to test hypotheses about whether a response variable is caused by or correlated with one or more traits or variables that can be measured (Fisher & vanBelle, supra, Ch. 10).

From the analyses described above, a mathematical model may be readily constructed by the skilled artisan that predicts clinical response as a function of genotype or haplotype content.

The identification of an association between a clinical response and a genotype or haplotype (or haplotype pair) for the gene may be the basis for designing a diagnostic method to determine those individuals who will or will not respond to the treatment, or alternatively, will respond at a lower level and thus may require more treatment, *i. e.*, a greater dose of a drug. The diagnostic method may take one of several forms: for example, a direct DNA test (*i.e.*, genotyping or haplotyping one or more of the polymorphic sites in the gene), a serological test, or a physical exam measurement. The only requirement is that there be a good correlation between the diagnostic test results and the underlying genotype or haplotype that is in turn correlated with the clinical response. In a preferred embodiment, this diagnostic method uses the predictive haplotyping method described above.

A computer may implement any or all analytical and mathematical operations involved in practicing the methods of the present disclosure. In addition, the computer may execute a program that generates views (or screens) displayed on a display device and with which the user can interact to view and analyze large amounts of information relating to the gene and its genomic variation, including chromosome location, gene structure, and gene family, gene expression data, polymorphism data, genetic sequence data, and clinical data population data (*e.g.*, data on ethnogeographic origin, clinical responses, genotypes, and haplotypes for one or more populations). The polymorphism data described herein may be stored as part of a relational database (*e.g.*, an instance of an Oracle database or a set of ASCII flat files). These polymorphism data may be stored on the computer's hard drive or may, for example, be stored on a CD-ROM or on one or more other storage devices accessible by the computer. For example, the data may be stored on one or more databases in communication with the computer via a network.

In other embodiments, the disclosure provides methods, compositions, and kits for haplotyping and/or genotyping the gene in an individual. The compositions contain oligonucleotide probes and primers designed to specifically hybridize to one or more target regions containing, or that are adjacent to, a polymorphic site. The methods and compositions for establishing the genotype or haplotype of an individual at the novel polymorphic sites described herein are useful for studying the effect of the polymorphisms in the etiology of diseases affected by the expression and function of the protein, studying the efficacy of drugs targeting, predicting individual susceptibility to diseases affected by the expression and function of the protein and predicting individual responsiveness to drugs targeting the gene product.

In yet another embodiment, the disclosure provides a method for identifying an association between a genotype or haplotype and a trait. In preferred embodiments, the trait is susceptibility to a disease, severity of a disease, the staging of a disease or response to a drug. Such methods have applicability in developing diagnostic tests and therapeutic treatments for all pharmacogenetic applications where there is the potential for an association between a genotype and a treatment outcome including efficacy measurements, PK measurements and side effect measurements.

The disclosure also provides a computer system for storing and displaying polymorphism data determine for the gene. The computer system comprises a computer processing unit; a display; and a database containing the polymorphism data. The polymorphism data includes the polymorphisms, the genotypes and the haplotypes identified for the gene in a reference population. In a preferred embodiment, the computer system is capable of producing a display showing haplotypes organized according to their evolutionary relationships.

In another aspect, the disclosure provides SNP probes, which are useful in classifying people according to their types of genetic variation. The SNP probes are oligonucleotides, which can discriminate between alleles of a SNP nucleic acid in conventional allelic discrimination assays.

As used herein, a "SNP nucleic acid" is a nucleic acid sequence, which comprises a nucleotide that is variable within an otherwise identical nucleotide sequence between individuals or groups of individuals, thus, existing as alleles. Such SNP nucleic acids are preferably from about 15 to about 500 nucleotides in length. The SNP nucleic acids may be part of a chromosome, or they may be an exact copy of a part of a chromosome, *e.g.*, by amplification of such a part of a chromosome through PCR or through cloning. The SNP nucleic acids are referred to hereafter simply as "SNPs". The SNP probes are oligonucleotides that are complementary to a SNP nucleic acid.

As used herein, the term "complementary" means exactly complementary throughout the length of the oligonucleotide in the Watson and Crick sense of the word.

In certain preferred embodiments, the oligonucleotides are complementary to one allele of the SNP nucleic acid, but not to any other allele of the SNP nucleic acid. Oligonucleotides according to this embodiment can discriminate between alleles of the SNP nucleic acid in various ways. For example, under stringent hybridization conditions, an oligonucleotide of appropriate length will hybridize to one allele of the SNP nucleic acid, but not to any other allele of the SNP nucleic acid. The oligonucleotide may be labelled by a radiolabel or a fluorescent label. Alternatively, an oligonucleotide of appropriate length can be used as a primer for PCR, wherein the 3' terminal nucleotide is complementary to one allele of the SNP nucleic acid, but not to any other allele. In this embodiment, the presence or absence of amplification by PCR determines the haplotype of the SNP nucleic acid.

Genomic and cDNA fragments comprise at least one novel polymorphic site identified herein and have a length of at least 10 nucleotides and may range up to the full length of the gene. Preferably, a fragment according to the present disclosure is between 100 and 3000 nucleotides in length, and more preferably between 200 and 2000 nucleotides in length, and most preferably between 500 and 1000 nucleotides in length.

In describing the polymorphic sites identified herein reference is made to the sense strand of the gene for convenience. However, as recognized by the skilled artisan, nucleic acid molecules containing the gene may be complementary double stranded molecules and thus reference to a particular site on the sense strand refers as well to the corresponding site on the complementary antisense strand. Thus, reference may be made to the same polymorphic site on either strand and an oligonucleotide may be designed to hybridize specifically to either strand at a target region containing the polymorphic site. Thus, the disclosure also includes single-stranded polynucleotides that are complementary to the sense strand of the genomic variants described herein.

In a preferred embodiment, such kit may further comprise a DNA sample collecting means.

In particular, the genotyping primer composition may comprise at least two sets of allele specific primer pairs. Preferably, the two genotyping oligonucleotides are packaged in separate containers.

It is to be understood that the methods described herein generally may further comprise the use of a kit. The methods of the invention are performed *ex-vivo*.

Effect(s) of the polymorphisms identified herein on expression of may be investigated by preparing recombinant cells and/or organisms, preferably recombinant animals, containing a polymorphic variant of the gene. As used herein, "expression" includes but is not limited to one or more of the following: transcription of the gene into precursor mRNA; splicing and other processing of the precursor mRNA to produce mature mRNA; mRNA stability; translation of the mature mRNA into protein (including codon usage and tRNA availability); and glycosylation and/or other modifications of the translation product, if required for proper expression and function.

To prepare a recombinant cell, the desired isogene may be introduced into the cell in a vector such that the isogene remains extrachromosomal. In such a situation, the gene will be expressed by the cell from the extrachromosomal location. In a preferred embodiment, the isogene is introduced into a cell in such a way that it recombines with the endogenous gene present in the cell. Such recombination requires the occurrence of a double recombination event, thereby resulting in the desired gene polymorphism. Vectors for the introduction of genes both for recombination and for extrachromosomal maintenance are known in the art, and any suitable vector or vector construct may be used in the invention. Methods such as electroporation, particle bombardment, calcium phosphate co-precipitation and viral transduction for introducing DNA into cells are known in the art; therefore, the choice of method may lie with the competence and preference of the skilled practitioner.

Recombinant organisms, i.e., transgenic animals, expressing a variant gene are prepared using standard procedures known in the art. Preferably, a construct comprising the variant gene is introduced into a nonhuman animal or an ancestor of the animal at an embryonic stage, i.e., the one-cell stage, or generally not later than about the eight-cell stage. Transgenic animals carrying the constructs can be made by several methods known to those having skill in the art. One method involves transfecting into the embryo a retrovirus constructed to contain one or more insulator elements, a gene or genes of interest, and other components known to those skilled in the art to provide a complete shuttle vector harbouring the insulated gene(s) as a transgene, *see e.g.,* U.S. Pat. No. 5,610,053. Another method involves directly injecting a transgene into the embryo. A third method involves the use of embryonic stem cells.

Examples of animals, into which the isogenes may be introduced include, but are not limited to, mice, rats, other rodents, and nonhuman primates (see "The Introduction of Foreign Genes into Mice" and the cited references therein, In: Recombinant DNA, Eds. J .D. Watson, M. Gilman, J. Witkowski, & M. Zoller; W.H. Freeman and Company, New York, pages 254-272). Transgenic animals stably expressing a human isogene and producing human protein can be used as biological models for studying diseases related to abnormal expression and/or activity, and for screening and assaying various candidate drugs, compounds, and treatment regimens to reduce the symptoms or effects of these diseases.

In practicing the present invention, many conventional techniques in molecular biology, microbiology and recombinant DNA are used. These techniques are well-known and are explained in, *e.g., "*Current Protocols in Molecular Biology", Vols. I-III, Ausubel, Ed. (1997); Sambrook et al., "Molecular Cloning: A Laboratory Manual", 2nd Ed., Cold Spring Harbor Laboratory Press, Cold Spring Harbor, NY (1989); "DNA Cloning: A Practical Approach", Vols. I and II, Glover, Ed. (1985); "Oligonucleotide Synthesis", Gait, Ed. (1984); "Nucleic Acid Hybridization", Hames & Higgins, Eds. (1985); "Transcription and Translation", Hames & Higgins, Eds. (1984); "Animal Cell Culture", Freshney, Ed. (1986); "Immobilized Cells and Enzymes", IRL Press (1986); Perbal, "A Practical Guide to Molecular Cloning"; the series, Methods in Enzymol., Academic Press, Inc. (1984); "Gene Transfer Vectors for Mammalian Cells", Miller and Calos, Eds., Cold Spring Harbor Laboratory, NY (1987); and Methods in Enzymology, Vols. 154 and 155, Wu & Grossman, and Wu, Eds., respectively.

The standard control levels of the gene expression product, thus determined in the different control groups, would then be compared with the measured level of an gene expression product in a given patient. This gene expression product could be the characteristic mRNA associated with that particular genotype group or the polypeptide gene expression product of that genotype group. The patient could then be classified or assigned to a particular genotype group based on how similar the measured levels were compared to the control levels for a given group.

As one of skill in the art will understand, there will be a certain degree of uncertainty involved in making this determination. Therefore, the standard deviations of the control group levels would be used to make a probabilistic determination and the methods of this disclosure would be applicable over a wide range of probability based genotype group determinations. Thus, for example and not by way of limitation, in one embodiment, if the measured level of the gene expression product falls within 2.5 standard deviations of the mean of any of the control groups, then that individual may be assigned to that genotype group. In another embodiment if the measured level of the gene expression product falls within 2.0 standard deviations of the mean of any of the control groups then that individual may be assigned to that genotype group. In still another embodiment, if the measured level of the gene expression product falls within 1.5 standard deviations of the mean of any of the control groups then that individual may be assigned to that genotype group. In yet another embodiment, if the measured level of the gene expression product is 1.0 or less standard deviations of the mean of any of the control groups levels then that individual may be assigned to that genotype group.

Thus this process will allow the determining, with various degrees of probability, which group a specific patient should be place in and such assignment to a genotype group would then determine the risk category into which the individual should be placed.

Methods to detect and measure mRNA levels and levels of polypeptide gene expression products are well known in the art and include the use of nucleotide microarrays and polypeptide detection methods involving mass spectrometers and/or antibody detection and quantification techniques. See also, Human Molecular Genetics, 2nd Edition. Tom Strachan & Andrew, Read (John Wiley and Sons, Inc. Publication, NY, 1999).

Furthermore, detection of the concentration of the polypeptide (protein) expression product of the gene in body fluids or tissues can be used to determine the presence or absence of the polymorphism, and the relative level of the polypeptide expression product can be used to determine if the polymorphism is present in a homozygous or heterozygous state and therefore the risk category of the individual.

As used herein, "medical condition" includes, but is not limited to, any condition or disease manifested as one or more physical and/or psychological symptoms for which treatment is desirable, and includes previously and newly-identified diseases and other disorders.

As used herein the term "polymorphism" shall mean any sequence variant present at a frequency of >1% in a population. The sequence variant may be present at a frequency significantly greater than 1% such as 5% or 10 % or more. Also, the term may be used to refer to the sequence variation observed in an individual at a polymorphic site. Polymorphisms include nucleotide substitutions, insertions, deletions and microsatellites and may, but need not, result in detectable differences in gene expression or protein function.

As used herein, the term "clinical response" means any or all of the following: a quantitative measure of the response, no response and adverse response, *i.e.*, side effects.

As used herein the term "allele" shall mean a particular form of a gene or DNA sequence at a specific chromosomal location (locus).

As used herein, the term "genotype" shall mean an unphased 5' to 3' sequence of nucleotide pair(s) found at one or more polymorphic sites in a locus on a pair of homologous chromosomes in an individual. As used herein, genotype includes a full-genotype and/or a sub-genotype.

As used herein, the term "polynucleotide" shall mean any RNA or DNA, which may be unmodified or modified RNA or DNA. Polynucleotides include, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, polynucleotide refers to triple-stranded regions comprising RNA or DNA or both RNA and DNA. The term polynucleotide also includes DNAs or RNAs containing one or more modified bases and DNAs or RNAs with backbones modified for stability or for other reasons.

As used herein the term "gene" shall mean a segment of DNA that contains all the information for the regulated biosynthesis of an RNA product, including promoters, exons, introns, and other untranslated regions that control expression.

As used herein the term "polypeptide" shall mean any polypeptide comprising two or more amino acids joined to each other by peptide bonds or modified peptide bonds, i.e., peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well known in the art. Such modifications are well described in basic texts and in more detailed monographs, as well as in a voluminous research literature.

As used herein, the term "polymorphic site" shall mean a position within a locus at which at least two alternative sequences are found in a population, the most frequent of which has a frequency of no more than 99%.

As used herein, the term "nucleotide pair" shall mean the nucleotides found at a polymorphic site on the two copies of a chromosome from an individual.

As used herein, the term "phased" means, when applied to a sequence of nucleotide pairs for two or more polymorphic sites in a locus, the combination of nucleotides present at those polymorphic sites on a single copy of the locus is known.

In order to deduce a correlation between clinical response to a treatment and a genotype or haplotype, it is necessary to obtain data on the clinical responses exhibited by a population of individuals who received the treatment, hereinafter the "clinical population". This clinical data may be obtained by analyzing the results of a clinical trial that has already been run and/or the clinical data may be obtained by designing and carrying out one or more new clinical trials.

As used herein, the term "clinical trial" means any research study designed to collect clinical data on responses to a particular treatment, and includes, but is not limited to, Phase I, II and III clinical trials. Standard methods are used to define the patient population and to enroll subjects.

As used herein the term "locus" shall mean a location on a chromosome or DNA molecule corresponding to a gene or a physical or phenotypic feature.

The therapeutic treatment of interest is administered to each individual in the trial population and each individual's response to the treatment is measured using one or more predetermined criteria. It is contemplated that in many cases, the trial population will exhibit a range of responses and that the investigator will choose the number of responder groups, e.g., low, medium and high, made up by the various responses. In addition, the gene for each individual in the trial population is genotyped and/or haplotyped, which may be done before or after administering the treatment.

*The Detection of Nucleic Acids and Proteins as Markers.* In a particular embodiment, the level of mRNA corresponding to the marker can be determined both by *in situ* and by *in vitro* formats in a biological sample using methods known in the art. The term "biological sample" is intended to include tissues, cells, biological fluids and isolates thereof, isolated from a subject, as well as tissues, cells and fluids present within a subject. Many expression detection methods use isolated RNA. For *in vitro* methods, any RNA isolation technique that does not select against the isolation of mRNA can be utilized for the purification of RNA from cells. See, *e.g.*, Ausubel et al., Ed., Curr. Prot. Mol. Biol., John Wiley & Sons, NY (1987-1999). Additionally, large numbers of tissue samples can readily be processed using techniques well-known to those of skill in the art, such as, e.g., the single-step RNA isolation process of U.S. Pat. No. 4,843,155.

The isolated mRNA can be used in hybridization or amplification assays that include, but are not limited to, Southern or Northern analyses, PCR analyses and probe arrays. One preferred diagnostic method for the detection of mRNA levels involve contacting the isolated mRNA with a nucleic acid molecule (probe) that can hybridize to the mRNA encoded by the gene being detected. The nucleic acid probe can be, e.g., a full-length cDNA, or a portion thereof, such as an oligonucleotide of at least 7, 15, 30, 50, 100, 250 or 500 nucleotides in length and sufficient to specifically hybridize under stringent conditions to a mRNA or genomic DNA encoding a marker of the present disclosure. Other suitable probes for use in the diagnostic assays of the disclosure are described herein. Hybridization of an mRNA with the probe indicates that the marker in question is being expressed.

In one format, the mRNA is immobilized on a solid surface and contacted with a probe, for example, by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in an Affymetrix gene chip array. A skilled artisan can readily adapt known mRNA detection methods for use in detecting the level of mRNA encoded by the markers of the present disclosure.

An alternative method for determining the level of mRNA in a sample involves the process of nucleic acid amplification, e.g., by RT-PCR (the experimental embodiment set forth in Mullis, U.S. Pat. No. 4,683,202 (1987); ligase chain reaction, Barany (1991), *supra*; self-sustained sequence replication, Guatelli et al., Proc. Natl. Acad. Sci. USA, Vol. 87, pp. 1874-1878 (1990); transcriptional amplification system, Kwoh et al., Proc. Natl. Acad. Sci. USA, Vol. 86, pp. 1173-1177 (1989); Q-Beta Replicase, Lizardi et al., Biol. Technology, Vol. 6, p. 1197 (1988); rolling circle replication, U.S. Pat. No. 5,854,033 (1988); or any other nucleic acid amplification method, followed by the detection of the amplified molecules using techniques well-known to those of skill in the art. These detection schemes are especially useful for the detection of the nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10-30 nucleotides in length and flank a region from about 50-200 nucleotides in length. Under appropriate conditions and with appropriate reagents, such primers permit the amplification of a nucleic acid molecule comprising the nucleotide sequence flanked by the primers.

For *in situ* methods, mRNA does not need to be isolated form the cells prior to detection. In such methods, a cell or tissue sample is prepared/processed using known histological methods. The sample is then immobilized on a support, typically a glass slide, and then contacted with a probe that can hybridize to mRNA that encodes the marker.

As an alternative to making determinations based on the absolute expression level of the marker, determinations may be based on the normalized expression level of the marker. Expression levels are normalized by correcting the absolute expression level of a marker by comparing its expression to the expression of a gene that is not a marker, e.g., a housekeeping gene that is constitutively expressed. Suitable genes for normalization include housekeeping genes, such as the actin gene or epithelial cell-specific genes. This normalization allows the comparison of the expression level in one sample, e.g., a patient sample, to another sample or between samples from different sources.

Alternatively, the expression level can be provided as a relative expression level. To determine a relative expression level of a marker, the level of expression of the marker is determined for 10 or more samples of normal versus disease biological samples, preferably 50 or more samples, prior to the determination of the expression level for the sample in question. The mean expression level of each of the genes assayed in the larger number of samples is determined and this is used as a baseline expression level for the marker. The expression level of the marker determined for the test sample (absolute level of expression) is then divided by the mean expression value obtained for that marker. This provides a relative expression level.

Preferably, the samples used in the baseline determination will be from patients who do not have the polymorphism. The choice of the cell source is dependent on the use of the relative expression level. Using expression found in normal tissues as a mean expression score aids in validating whether the marker assayed is specific (versus normal cells). In addition, as more data is accumulated, the mean expression value can be revised, providing improved relative expression values based on accumulated data.

*Detection of Polypeptides.* In another embodiment of the present disclosure, a polypeptide corresponding to a marker is detected. A preferred agent for detecting a polypeptide is an antibody capable of binding to a polypeptide corresponding to a marker, preferably an antibody with a detectable label. Antibodies can be polyclonal, or more preferably, monoclonal. An intact antibody, or a fragment thereof, *e.g.*, Fab or $F(ab')_2$ can be used. The term "labelled", with regard to the probe or antibody, is intended to encompass direct-labelling of the probe or antibody by coupling, i.e., physically linking, a detectable substance to the probe or antibody, as well as indirect-labelling of the probe or antibody by reactivity with another reagent that is directly-labelled. Examples of indirect labelling include detection of a primary antibody using a fluorescently-labelled secondary antibody and end-labelling of a DNA probe with biotin such that it can be detected with fluorescently-labelled streptavidin.

Proteins from individuals can be isolated using techniques that are well-known to those of skill in the art. The protein isolation methods employed can, e.g., be such as those described in Harlow & Lane (1988), *supra.*

A variety of formats can be employed to determine whether a sample contains a protein that binds to a given antibody. Examples of such formats include, but are not limited to, EIA; radioimmunoasay (RIA), Western blot analysis and ELISA. A skilled artisan can readily adapt known protein/antibody detection methods for use in determining whether cells express a marker and the relative concentration of that specific polypeptide expression product in blood or other body tissues.

In one format, antibodies or antibody fragments, can be used in methods, such as Western blots or immunofluorescence techniques to detect the expressed proteins. In such uses, it is generally preferable to immobilize either the antibody or proteins on a solid support. Suitable solid phase supports or carriers include any support capable of binding an antigen or an antibody. Well-known supports or carriers include glass, polystyrene, polypropylene, polyethylene, dextran, nylon, amylases, natural and modified celluloses, polyacrylamides, gabbros and magnetite.

One skilled in the art will know many other suitable carriers for binding antibody or antigen, and will be able to adapt such support. For example, protein isolated from patient cells can be run on a polyacrylamide gel electrophoresis and immobilized onto a solid phase support, such as nitrocellulose. The support can then be washed with suitable buffers followed by treatment with the detectably-labelled antibody. The solid phase support can then be washed with the buffer a second time to remove unbound antibody. The amount of bound label on the solid support can then be detected by conventional means and this measurement translated into a level or concentration of protein in blood or another body tissue.

The disclosure also encompasses kits for detecting the presence of a polypeptide or nucleic acid corresponding to a marker in a biological sample, e.g., any body fluid including, but not limited to, serum, plasma, lymph, cystic fluid, urine, stool, csf, acitic fluid or blood and including biopsy samples of body tissue. For example, the kit can comprise a labelled compound or agent capable of detecting a polypeptide or an mRNA encoding a polypeptide corresponding to a marker in a biological sample and means for determining the amount of the polypeptide or mRNA in the sample, *e.g.*, an antibody which binds the polypeptide or an oligonucleotide probe which binds to DNA or mRNA encoding the polypeptide. Kits can also include instructions for interpreting the results obtained using the kit.

For antibody-based kits, the kit can comprise, *e.g.*, 1) a first antibody, e.g., attached to a solid support, which binds to a polypeptide corresponding to a marker; and, optionally; and optionally 2) a second, different antibody which binds to either the polypeptide or the first antibody and is conjugated to a detectable label.

For oligonucleotide-based kits, the kit can comprise, *e.g.*, 1) an oligonucleotide, *e.g.*, a detectably-labelled oligonucleotide, which hybridizes to a nucleic acid sequence encoding a polypeptide corresponding to a marker; or 2) a pair of primers useful for amplifying a nucleic acid molecule corresponding to a marker.

The kit can also comprise, *e.g.*, a buffering agent, a preservative or a protein-stabilizing agent. The kit can further comprise components necessary for detecting the detectable-label, *e.g.*, an enzyme or a substrate. The kit can also contain a control sample or a series of control samples, which can be assayed and compared to the test sample. Each component of the kit can be enclosed within an individual container and all of the various containers can be within a single package, along with instructions for interpreting the results of the assays performed using the kit.

*Kits.* The kits may contain a written product on or in the kit container. The written product describes how to use the reagents contained in the kit to determine whether a patient will experience hepatotoxicity during drug treatment. In several embodiments, the use of the reagents can be according to the methods of the invention. In one embodiment, the reagents are primer pairs for performing PCR analysis of *IL1A* genetic polymorphisms.

EXAMPLE

CLINICAL PHARMACOGENETIC ANALYSIS OF HEPATOTOXICITY IN THE CLINICAL TRIAL

*Demographics of clinical pharmacogenetic analysis participants.* Of the 139 subjects enrolled in the clinical trial, 83 consented to participation in the clinical pharmacogenetic portion of the clinical trial. This represents about 60% of the total population that participated in the clinical trial. The clinical pharmacogenetic analysis population was representative of the clinical trial group in terms of age, race and gender. Furthermore, the consent rate was comparable for each arm of the trial (placebo, 50, 100 and 150 mg/day), such that the clinical pharmacogenetic analysis was not biased toward one dosage group. No statistically significant differences were observed between the demographics of the clinical pharmacogenetic population compared to the overall trial population.

TABLE 1

Distribution of clinical pharmacogenetic samples compared to the overall clinical trial samples

| | | CPG samples | Trial samples |
|---|---|---|---|
| *AGE (mean,years)[a]* | | 58.8 | 59.3 |
| *RACE[b]* | | | |
| | *Caucasian* | (70) 84% | (117) 84% |
| | *Black* | (5) 6% | (9) 6.4% |
| | *Oriental* | (1) 1.2% | (2) 1.4% |
| | *Hispanic* | (7) 8.4% | (9) 7% |
| | *Other* | (0) 0% | (2) 1.4% |
| *GENDER[c]* | | | |
| | *Male* | (50) 60% | (88) 63% |
| | *Female* | (33) 40% | (51) 37% |
| *DOSE[d]* | | | |
| | *0 mg/day* | (23) 28% | (34) 24% |
| | *50 mg/day* | (20) 24% | (32) 23% |
| | *100 mg/day* | (20) 24% | (37) 27% |
| | *150 mg/day* | (20) 24% | (36) 26% |
| *Hepatotoxicity[e]* | | | |
| | *Yes* | (6) 7% | (9) 6% |
| | *No* | (77)93% | (130)94% |

[a]$p<0.7748$ (ANOVA)
[b]$p<0.9112$ (Fisher's Exact)
[c]$p<0.6698$ (Fisher's Exact)
[d]$p<0.9342$ (Fisher's Exact)
[e]$p<1.000$ (Fisher's Exact)

Blood samples from each patient were collected at individual trial sites and shipped to Covance (Geneva, Switzerland), where genomic DNA was extracted using the PUREGENE™ DNA Isolation Kit (D-50K) (Gentra, Minneapolis, MN).

*Genotyping.* A total of 18 loci in seven genes were genotyped. SNP assays were designed using information from public databases such as OMIM, the SNP Consortium, Locus Link and dbSNP, and information from Third Wave Technologies, Inc. (TWT, Madison, WI). Any loci that were not polymorphic in this trial population were not analyzed further. Genotyping was performed with 40-60 ng of genomic DNA using the Invader® assay developed by Third Wave Technologies according to the manufacturer's instructions. Lyamichev V et al.,Nat Biotechnol 17: 292-6 (1999); Ryan D et al.. Mol Diagn 4: 135-44 9 (1999)

Each SNP interrogated in this trial was assigned a clinical pharmacogenetic (CPG) identifier, referred to as the PG locus ID. See TABLE 2 for a list of all SNPs assayed in this trial, along with their PG locus IDs and details about the location of the polymorphism within the gene of interest.

TABLE 2
List of genes examined in the clinical pharmacogenetic analysis

| Gene Symbol | Gene Name | CPG Locus ID | REF ACC | Allele 1 Freq. | Allele 2 Freq. | Position | Location |
|---|---|---|---|---|---|---|---|
| ABCB1 | ATP-binding cassette, sub family B (MDR/ TAP), member 1 | 181 | M29445 0.47 | | 0.53 | 176 | Exon 26 ILE1144IL E |
| ABCB1 | ATP-binding cassette, sub-family B (MDR/ TAP), member 1 | 1006 | AC005068.1 | 0.63 | 0.37 | 83646 | Unknown |
| ABCB1 | ATP-binding cassette, sub-family B (MDR/ TAP), member 1 | 1045 | AC002457.1 | 0.48 | 0.52 | 19981202 | Unknown |
| CD14 | CD14 antigen | 1708 | U00699 | 0.56 | 0.44 | 312 | Promoter |
| CYP2D6 | cytochronte P450, subfamily IID (debrisoquine, sparteine, etc., -metabolizing), polypeptide 6 | 31 | M33388 | 0.86* | 0.14* | 3465 | Intron |
| CYP2D6 | cytochrome P450, subfamily IID (debrisoquine, sparteine, etc., -metabolizing), polypeptide 6 | 211 | M33388 | 0.60* | 0.40* | 4469 | Exon 6 ARG269CY S |
| CYP2D6 | cytochrome P450, subfamily IID (debrisoquine, sparteine, etc., -metabolizing), polypeptide 6 | 212 | M33388 | 0.52 | 0.48 | 5799 | Exon 9 THR486SE R |
| CYP2D6 | cytochrome P450, subfamily IID (debrisoquine, sparteine, etc., -metabolizing), polypeptide 6 | 213 | M33388 | 0.83 | 0.17 | 1719 | Exon 1 PRO34SER |
| CYP3A4 | cytochrome P450, subfamily IIIA (niphedipine oxidase), polypeptide 4 | 2325 | AF209389 | 0.84* | 0.16* | 20338 | Intron 10 |
| IL1 | interleukin 1, alpha | 279 | X03833 | 0.72* | 0.28* | 549 | Promoter |

(continued)

List of genes examined in the clinical pharmacogenetic analysis

| Gene Symbol | Gene Name | CPG Locus ID | REF ACC | Allele 1 Freq. | Allele 2 Freq. | Position | Location |
|---|---|---|---|---|---|---|---|
| IL1A | interleukin 1, alpha | 302 | X03833 | 0.72* | 0.28* | 6282 | Exon 5 |
| ILIA+ | interleukin 1, alpha | 303 | X03833 | 1.00 | 0.00 | 4282 | Exon 4 |
| NR1I2 | nuclear receptor subfamily 1, group I, member 2 | 2641 | AC069444 | 0.62* | 0.38* | 42813 | Promoter |
| NR1I2 | nuclear receptor subfamily 1, group I, member 2 | 2642 | AF061056 | 0.60 | 0.40 | 252 | Intron |
| NR1I2 | nuclear receptor subfamily 1, group I, member 2 | 2643 | AF061056 | 0.80 | 0.20 | 11156 | 3' UTR |
| ORM1 | orosomucoid 1 (alpha-1-acid glycoprotein) | 1831 | NT_031830 | 0.57* | 0.43* | 191 | Exon 1 |
| ORM1 | orosomucoid I (alpha-1-acid glycoprotein) | 1832 | NT_031830 | 0.97 | 0.03 | 2077 | Exon 5 |
| ORM1 | orosomucoid I (alpha-1-acid glycoprotein) | 1833 | NT_031830 | 0.96 | 0.04 | 5041 | Intron |

*This SNP is not in Hardy-Weinberg equilibrium in this patient population.

+This locus is not polymorphic in these patient populations and was not used in the analysis.

Loci in *ABCB1*, *CD14*, *ILIA,* and *ORM1* were interrogated by directly assaying genomic DNA using Third Wave Technologies technology. Because of high sequence homology within the CYP450 family of genes, *CYP2D6* was polymerase chain reaction (PCR) amplified in three fragments prior to Invader® assay genotyping to insure specificity. Primer sequences for each segment are listed in TABLE 3 along with the region of the gene spanned by each primer set. Each amplicon was generated in a 25 $\mu$l reaction containing 20-60 ng of genomic DNA, 0.5 $\mu$l of 10 mM dNTPs, 2.5 $\mu$l of 10X PCR Buffer I with 15 mM MgCl$_2$ (Applied Biosystems, Foster City, CA), 2.5 $\mu$l DMSO, 0.5 $\mu$l of 20 $\mu$M CYP2D6-forward primer, 0.5 $\mu$l of 20 $\mu$M CYP2D6-reverse primer, and 1.25 U Taq DNA polymerase (Applied Biosystems). 35 cycles of amplification were performed using the following conditions: 94°C, 30 sec; 65°C, 1 min.; 72°C, 2 min. Amplification of the appropriate product was confirmed on five random samples by fractionation on a 1% agarose gel containing ethidium bromide. Amplicons were diluted 1:10 in TE (pH 8.0) before genotyping.

TABLE 3

Primers used to amplify CYP2D6

| Gene region (PG locus ID) | Primer Name | Primer Sequence (5'→3') | Size (bp) |
|---|---|---|---|
| Exons 1 and 2 (31) | 2D6L1F1 | CTGGGCTGGGAGCAGCCTC (SEQ ID NO:5) | 2036 |
| | 2D6L1R1 | CACTCGCTGGCCTGTTTCATGTC (SEQ ID NO:6) | |
| Exons 3, 4 and 5 (31 & 211) | 2D6L2F | CTGGAATCCGGTGTCGAAGTGG (SEQ ID NO:7) | 1683 |
| | 2D6L2R2 | CTCGGCCCCTGCACTGTTTC (SEQ ID NO:8) | |
| Exons 7, 8 and 9 (212) | 2D6L3F | GAGGCAAGAAGGAGTGTCAGGG (SEQ ID NO:9) | 1754 |
| | 2D6L3R5B | AGTCCTGTGGTGAGGTGACGAGG (SEQ ID NO:10) | |

PCR for the *CYP3A4* gene was performed in a 25 $\mu$l reaction containing 15-30 ng genomic DNA, 0.4 $\mu$l of 10 mM dNTPs, 2.5 $\mu$l of 10X PCR BufferI with 15 mM MgCl$_2$ (Applied Biosystems), 0.75 $\mu$l of 20 $\mu$M CYP3A4-forward primer, 0.75 $\mu$l of 20 $\mu$M CYP3A4-reverse primer, and 0.75 U Taq DNA polymerase (Applied Biosystems). 30 cycles of amplification were performed using the following conditions: 94˚C, 30 sec; 58˚C, 30 sec; 72˚C, 30 sec. To confirm that the appropriate product was generated, five samples were fractionated on a 1% agarose gel containing ethidium bromide and fragment size was visualized. Primer sequences are as follows:

CYP3A4Exon10F-(5'-TGGATGGCCCACATTCTCG-3'; SEQ ID NO:11), and
CYP3A4Exon10R-(5' CTTCCTACATAGAGTCAGTG-3'; SEQ ID NO:12). A 1:20 dilution of the PCR product in TE (pH 8.0) was run against PG locus ID 2325 using a 384 well biplex plate for amplified DNA.

Restriction Fragment Length Polymorphism (RFLP) analysis was used to genotype three polymorphic loci in the *NR1I2*. *NR1I2* sequences were first PCR amplified in 25 $\mu$l reactions containing 15-30 ng genomic DNA, 0.4 $\mu$l of 10 mM dNTPs, 2.5 $\mu$l of 10X PCR Buffer I with 15 mM MgCl$_2$ (Applied Biosystems), 0.50 $\mu$l of 20 $\mu$M NR1I2-forward primer, 0.50 $\mu$l of 20 $\mu$M NR1I2-reverse primer, and 0.75 U Taq DNA polymerase (Applied Biosystems). Sequences for the primer sets used for each assay are listed in TABLE 4. Thirty-five cycles of amplification were performed using the following conditions: 94˚C, 30 sec; 60˚C, 30 sec; 72˚C, 30 sec. Amplicons were fractionated on a 3% agarose gel containing ethidium bromide.

TABLE 4

RFLP analysis of *NR1I2* polymorphisms

| PG locus ID (Assay Name) | Primer Name | Primer Sequence (5'-3') | Restriction Endonuclease | Frag Size (bp) / allele call |
|---|---|---|---|---|
| 2642 (PXR-252) | PXR-252-F | GGACACAGAGTCT GTTCCTGG (SEQ ID NO:13) | BSMB1 | 318/A 204 & 114/G |
| | PXR-252-R | GAAGATGAAGGA TTCCTCTGGG (SEQ ID NO:14) | | |
| 2643 (PXR-11156) | PXR-11156-F | GACAAGGCTACGC TGACAATCAG (SEQ ID NO:15) | DdeI | 342/C 193 & 149/A |
| | PXR-11156-R | GCTTGCGTATGTT TCTATTTCCAC (SEQ ID NO:16) | | |
| 2641 (PXR-24113) | PXR-24113-F | CGGAGCAAAGAA CTTACCACC (SEQ ID NO:17) | HphI | 253/G 195 & 58/A |
| | PXR-24113-R | TGCAGGACCAGAG AGCATCAG (SEQ ID NO:18) | | |

Restriction enzymes used for RFLP analysis of the PCR products are listed in TABLE 4 along with the fragment size they produced and the resultant allele call. All restriction enzymes were purchased from New England Biolabs, Beverly, MA. Reaction conditions were as follows: (1) BMSB1 digests were performed in a 20 $\mu$l reaction using 2 $\mu$l of 10X Buffer 3 (New England Biolabs), 8 $\mu$l amplified DNA, and 2U BSMB1 enzyme. Reaction mixtures were incubated for 4.5 hours at 55˚C; (2) DdeI digests were performed in a 20 $\mu$l reaction using 2 $\mu$l of 10X Buffer 3 (New England Biolabs), 8 $\mu$l amplified DNA, and 4 U of DdeI enzyme. Reactions were incubated for 17 hours at 37˚C; (3) HphI digests were as DdeI digests except 10X Buffer 4 (New England Biolabs) was used instead of 10X Buffer 3. Digested DNA was fractionated (10 $\mu$l) on a 3% agarose gel containing ethidium bromide and band size was visualized.

*Statistical Analysis.* Analysis of variance (ANOVA) and Fisher's Exact tests were used for the analysis of the effect of genotype and hepatotoxicity. All statistical analyses were performed using the SAS 8.02 software. To correct for multiple testing, the Bonferroni correction method was performed (see, below).

*Hepatotoxicity and N-benzoyl-staurosporine metabolism.* The relationship between the occurrence of hepatotoxicity in the clinical trial and the dose of N-benzoyl-staurosporine administered was examined. The percentage of subjects per dose group that experienced hepatotoxicity are as follows: 3% for 50 mg/day, 8% for 100 mg/day and 14% of those that took 150 mg/day. While most of the subjects that experienced liver toxicity took the highest dose of N-benzoyl-staurosporine, the association between of N-benzoyl-staurosporine dose and hepatotoxicity is not significant (p = 0.09; Fisher's Exact).

Pharmacokinetic assessments were done in the clinical trial, but the two major metabolites of N-benzoyl-staurosporine could not be detected in the blood of most subjects because they are strongly bound to the serum protein $\alpha_1$-acid glycoprotein (AGP). AGP is a heavily glycosylated protein that is synthesized primarily in the liver and functions as an acute phase response protein. Hochepied T et al., Cytokine Growth Factor Rev 14: 25-34 (2003); Israili ZH & Dayton PG, Drug Met Rev 33: 161-235 (2001). An analysis was done to see if AGP levels correlated with the occurrence of hepatotoxicity. The maximum AGP level from visit 3-5 was used for this analysis, and all participants in the clinical trial, not just those that consented to the pharmacogenetics analysis, were included. The mean maximum concentration of AGP among those that experienced hepatotoxicity was 109.7 mg/dl, which was significantly higher than 87.8 mg/dl, the mean maximum concentration for those that did not experience hepatotoxicity (p=0.046, ANOVA).

AGP is encoded by two genes, *ORM1* and *ORM2,* which are closely linked on chromosome 9q31-34.1. Webb GC et al., Cytogenet Cell Genet 47: 18-21 (1998). *ORM1* is highly polymorphic (Yuasa I et al., Hum Genet 99: 393-8 (1997)), and three SNPs in *ORM1* were interrogated in this trial (PG locus IDs 1831, 1832 and 1833). No association was seen between genotypes at the three *ORM1* loci examined and levels of AGP detected in the serum. See, TABLE 5.

TABLE 5

ORM1 SNPs do not associate with maximum AGP levels.

| PG locus ID | AGP Max (p value, ANOVA) |
|---|---|
| 1831 | 0.51 |
| 1832 | 0.07 |
| 1833 | 0.95 |

Furthermore, the *ORM1* SNPs interrogated in this analysis did not associate with hepatotoxicity. See, TABLE 6, below. To examine possible associations between the occurrence of hepatotoxicity and SNPs in *CYP2D6, CYP3A4, ABCB1* and *NR1I2* more thoroughly, each liver enzyme was examined independently. The maximum serum alanine aminotransferase and aspartate transaminase values recorded on visits 3-5 were analyzed using ANOVAs. No associations were found between SNPs in genes that contribute to the metabolism and distribution of N-benzoyl-staurosporine and the maximum elevation of either alanine aminotransferase or aspartate transaminase. No significant association was found between maximum alanine aminotransferase or aspartate transaminase levels and the *ORM1* polymorphisms.

TABLE 6

Association between hepatotoxicity, ALT Max and AST Max and 17 SNPs located in 6 different genes

| Gene | PG Locus ID | Overall Hepatotoxicity Fisher's Exact) | ALT Max (p value; call (p value; ANOVA ANOVA) | AST Max (p values; |
|---|---|---|---|---|
| ABCB1 | 181 | 0.59 | 0.55 | 0.32 |
| ABCB1 | 1006 | 1.00 | 0.63 | 0.24 |
| ABCB1 | 1045 | 0.50 | 0.35 | 0.09 |
| CD14 | 1708 | 1.00 | 0.87 | 0.90 |
| CYP2D6 | 31 | 1.00 | 0.99 | 0.63 |
| CYP2D6 | 211 | 1.00 | 0.85 | 0.66 |
| CYP2D6 | 212 | 0.46 | 0.16 | 0.52 |
| CYP2D6 | 213 | 0.22 | 0.17 | 0.59 |
| CYP3A4 | 2325 | 0.72 | 0.38 | 0.49 |
| AL1A | 279 | 0.23 | 0.37 | 0.031 |
| IL1A | 302 | 0.29 | 0.38 | 0.029 |
| NR1I2 | 2641 | 0.82 | 0.56 | 0.21 |

(continued)

Association between hepatotoxicity, ALT Max and AST Max and 17 SNPs located in 6 different genes

| Gene | PG Locus ID | Overall Hepatoxicity Fisher's Exact) | ALT Max (p value; call (p value; ANOVA ANOVA) | AST Max (p values; |
|------|-------------|--------------------------------------|----------------------------------------------|--------------------|
| NR1I2 | 2642 | 0.84 | 0.28 | 0.86 |
| NR1I2 | 2643 | 1.00 | 0.57 | 0.73 |
| ORM1 | 1831 | 0.61 | 0.72 | 0.76 |
| ORM1 | 1832 | 0.24 | 0.80 | 0.82 |
| ORM1 | 1833 | 1.00 | 0.96 | 0.64 |

In summary, the data in TABLES 5 and 6 did not provide evidence that the hepatotoxicity observed in the clinical trial was the result of varied exposure to N-benzoyl-staurosporine.

However, N-benzoyl-staurosporine is metabolized by *CYP3A4* and *CYP2D6,* and is a substrate of the p-glycoprotein pump, encoded by *ABCB1. CYP3A4* is not very polymorphic, and genetic variants rarely account for differences seen in the function of the protein. Spurdle AB et al., Pharmacogenetics 12: 355-66 (July 2002) and internal analysis. Also, transcription of *CYP3A4* is regulated by the pregnane X receptor, PXR (Goodwin B et al., Annu Rev Pharmacol Toxicol 42: 1-23 (2002)), which is encoded by a polymorphic gene called *NR1I2*. Accordingly, polymorphisms in the following genes that contribute to N-benzoyl-staurosporine metabolism were examined: *CYP2D6* (PG locus IDs 31, 211, 212, 213); *CYP3A4* (PG locus ID 2325); *NR1I2* (PG locus IDs 2641, 2642, 2643); and *ABCB1* (PG locus IDs 181, 1006, 1045). The occurrence of hepatotoxicity in the clinical trial was not found to be associated with any of the polymorphisms interrogated in these four genes.

*Hepatotoxicity and genes associated with idiosyncratic mechanisms of liver injury.* Hepatotoxicity observed during the clinical stages of drug development is often associated with levels of exposure to the drug. However, some subjects may experience liver enzyme elevations because something about their intrahepatic milieu (levels of cytokines or enzymes that neutralize free radicals, for example) promotes toxicity of the compound. These mechanisms of liver toxicity are referred to as "idiosyncratic". Finding an association between genetic polymorphisms and hepatotoxicity arising from idiosyncratic mechanisms is not very likely because hepatotoxicity is a relatively rare event that can be influenced by multiple signalling pathways.

*Hepatotoxicity and genes associated with inflammatory response.* Polymorphisms in two genes that could contribute to acute phase responses in the liver, *CD14* and *IL1A*, were examined in this trial. *CD14* regulates release of inflammatory cytokines from Kuppfer cells (Jarvelainen HA et al., Hepatology 33: 1148-53 (2001)) and *IL1A* is an important mediator of responses to tissue damaging agents. Ramadori G & Christ B, Semin Liver Dis 19: 141-55 (1999). Two polymorphic loci in *ILIA* were interrogated in this trial (PG locus IDs 279 and 302) and three loci were investigated in *CD14* (PG locus IDs 2641, 2642 and 2643). No associations were found between the loci we studied and the overall hepatotoxicity call in the clinical trial. See, TABLE 5.

Next, SNPs in *IL1A* and *CD14* were analyzed for their possible association with elevations in each liver enzyme independently. The maximum serum aspartate transaminase or alanine aminotransferase value recorded between visits 3 and 5 was associated with genotypes at the loci of interest in *1L1A* and *CD14* using an ANOVA. No associations were found between alanine aminotransferase and SNPs in *IL1A* or *CD14.*

However, both *IL1A* SNPs, PG locus IDs 279 and 302, were associated with the maximum serum aspartate transaminase value recorded on visits 3, 4, or 5 (p = 0.031 and 0.029, respectively). PG locus ID 279, a C→T transition in the promoter of *IL1A* (position 549 in GenBank accession number X03833), has been reported to be in linkage disequilibrium with PG locus ID 302. Jouvenne P et al., Eur Cytokine Netw 10: 33-6 (1999). Our findings support strong linkage between these two loci (99.99%). PG locus ID 302 is a G→T base change in exon 5 (position 6282 in GenBank accession number X03833) that results in an amino acid substitution of alanine to serine. For each of these SNPs, the TT genotype is rare; only two individuals are TT for PG locus ID 279 and 302. For this reason, we analyzed TT homozygous individuals together with the GT (PG locus ID 302) or CT (PG locus ID 279) heterozygotes. Thus, for PG locus ID 279, subjects with a CC genotype were compared with subjects with a T at that locus (either CT or TT). For PG locus ID 279, the average maximum serum aspartate transaminase value for CC individuals was 37.1 U/L, while T (CT and TT) individuals had a significantly lower average maximum serum aspartate transaminase value of 23.1 U/L (p=0.0089, ANOVA). Likewise, for PG locus ID 302, subjects who were GG were compared to Ts (subjects who were either GT or TT). When the data were grouped using this approach, a much stronger association was seen between genotype at each locus and the occurrence of hepatotoxicity. For PG locus ID 302, GG individuals had an average maximum serum aspartate transaminase level of 36.6 U/L, which was significantly higher than 22.9 U/L, the average maximum serum aspartate transaminase level for T (CT and TT) individuals (p= 0.0097, ANOVA). The scatterplots in FIG. 1 and FIG. 2 show that the subjects with the highest maximum serum aspartate transaminase values while taking N-benzoyl-staurosporine were CC at PG locus ID 279 and GG at PG locus ID 302.

Because multiple SNPs were tested for their association with hepatotoxicity in the clinical trial, a correction factor was applied to the results. The Bonferroni correction method requires that p values be adjusted by a factor of 17 (the number of polymorphic SNPs interrogated in this trial). $\text{Bonferroni} = \dfrac{0.05}{\eta} = \dfrac{0.05}{17} = 0.0029,$ where $\eta =$ *PCK412 _number_of_tests.* Using this correction factor, no SNP with an association with $p \geq 0.0029$ is significant. Associations between *IL1A* polymorphisms and serum aspartate transaminase levels had p values of 0.0089 and 0.0097.

In summary, subjects who are CC at PG locus 279 and GG at PG locus 302 within the *IL1A* gene are more likely to experience elevated serum aspartate transaminase levels after taking N-benzoyl-staurosporine. While the average aspartate transaminase maximum for CC subjects was not over the upper limit of normal, a scatterplot of the data demonstrates that all but one of the subjects that had serum aspartate transaminase levels over the upper limit of normal were CC for PG locus ID 279 (FIG. 1) and GG for PG locus ID 302 (FIG. 2).

Drug-induced liver toxicity is often characterized by inflammation. Jaeschke H et al., Toxicol Sci 65: 166-76 (2002). The interleukin 1 family of proteins is known to have multiple biological activities and are key regulators of the response to infection and injury. Like other proinflammatory cytokines, IL1A induces NF-κB activity, thereby increasing the transcription of cytokine-inducible genes. The effects of IL1A are mediated by the induction of other cytokines, including granulocyte colony-stimulating factor, tumour necrosis factor alpha (TNF), interleukin 6, interleukin 8, and platelet-derived growth factor. Paul WE, Fundamental Immunology, Fourth Edition (Lippingcott-Raven Publishers, Philadelphia, PA, 1999). Notably in mouse models, IL1A has been shown to act synergistically with TNF to induce liver injury. Nagakawa J et al., Immunopharmacol Immunotoxicol 13 : 485-98 (1991).

In addition to being associated with hepatotoxicity, the interleukin 1 family of proteins have been implicated in the induction of insulin secretion and stimulation of apoptosis in pancreatic β cells. Paul WE, Fundamental Immunology, Fourth Edition (Lippingcott-Raven Publishers, Philadelphia, PA, 1999). This is relevant for this trial since the clinical trial participants had either type I or type II diabetes. The production of IL1 by local inflammatory cells during the autoimmune process in insulin-dependent diabetes mellitus (type I diabetes) may contribute to the destruction of pancreatic β cells. Mandrup-Poulsen T et al., Cytokine 5: 185-81 (1993). Interleukin 1 family members have been shown to induce the production of nitric oxide (NO) by pancreatic islets, and reports in the literature support a role for NO in diabetes development. Furthermore, treatment of the pancreatic β-cell-like line RIN-5AH with IL1A resulted in apoptosis and necrosis within 4 hours after treatment. Vassiliadis S et al., Mediators Inflamm 8: 85-91 (1999). Interestingly, IL1A was shown to induce the expression of PKC in RIN-5AH cells by 30%. Therefore, in addition to influencing hepatotoxicity, IL1A may influence the efficacy of N-benzoyl-staurosporine in diabetic patients.

## Claims

1. Use of N-benzoyl-staurosporine in the manufacture of a medicament for the treatment of diabetic retinopathy with reduced hepatotoxicity in a selected patient population, wherein the patient population is selected on the basis of the genotype of the patients at an *IL1A* genetic locus predictive of hepatotoxicity, wherein the *IL1A* genetic locus predictive of hepatotoxicity genotype is:

   (i) a homozygous C genotype at the position 549 in the promoter of the *IL1A* gene as defined by the GenBank reference number X03833, and
   (ii) a homozygous G genotype at the position 6282 in exon 5 of the *IL1A* gene as defined by the GenBank reference number X03833.

2. A method for predicting N-benzoyl-staurosporine drug-induced hepatotoxicity in a subject, comprising the step of:

   determining *in vitro* the genotype of a subject at the *IL1A* genetic loci at ,

   (i) position 549 in the promoter of the *IL1A* gene as defined by the GenBank reference number X03833, and,
   (ii) position 6282 in exon 5 of the *IL1A* gene as defined by the GenBank reference number X03833

   wherein a homozygous C genotype at the position 549 in the promoter of the *IL1A* gene as defined by the GenBank reference number X03833, and a homozygous G genotype at the position 6282 in exon 5 of the *IL1A* gene as defined by the GenBank reference number X03833 is predictive of a risk of N-benzoyl-staurosporine drug-induced hepatoxicity.

## Patentansprüche

1. Verwendung von N-Benzoyl-staurosporin bei der Herstellung eines Medikaments zur Behandlung von diabetischer Retinopathie mit verringerter Hepatotoxizität bei einer ausgewählten Patientenpopulation, wobei die Patientenpopulation auf der Basis des Genotyps der Patienten an einem IL1A-Genort, der für Hepatotoxizität prädiktiv ist, ausgewählt ist, wobei der IL1A-Genort, der für den Hepatotoxizitätsgenotyp prädiktiv ist,

   (i) ein homozygoter C-Genotyp an der Position 549 im Promotor des iL1A-Gens gemäß Definition durch die Genbank-Referenznummer X03833 und
   (ii) ein homozygoter G-Genotyp an der Position 6282 im Exon 5 des IL1A-Gens gemäß Definition durch die Genbank-Referenznummer X03833 ist.

2. Verfahren zum Voraussagen einer durch das Arzneimittel N-Benzoyl-staurosporin induzierten Hepatotoxizität bei einem Patienten, das die folgenden Stufen umfasst:

   In-Vitro-Bestimmung des Genotyps eines Patienten an den IL1A-Genorten an

   (i) der Position 549 im Promotor des IL1A-Gens gemäß Definition durch die Genbank-Referenznummer X03833 und
   (ii) der Position 6282 im Exon 5 des IL1A-Gens gemäß Definition durch die Genbank-Referenznummer X03833,

   wobei ein homozygoter C-Genotyp an der Position 549 im Promotor des IL1A-Gens gemäß Definition durch die Genbank-Referenznummer X03833 und ein homozygoter G-Genotyp an der Position 6282 im Exon 5 des IL1A-Gens gemäß Definition durch die Genbank-Referenznummer X03833 prädiktiv für das Risiko einer durch das Arzneimittel N-Berzoyl-staurospohn induzierten Hepatotoxizität ist.

**Revendications**

1. Utilisation de N-benzoyl-staurosporine dans la fabrication d'un médicament destiné au traitement de la rétinopathie diabétique avec hépatotoxicité réduite chez une population sélectionnée de patients, où la population de patients est sélectionnée sur la base du génotype des patients au niveau d'un locus génétique d'IL1A prédictif d'hépatotoxicité, où le locus génétique d'IL1A prédictif du génotype d'hépatotoxicite est :

   (i) un génotype homozygote C en position 549 du promoteur du gène de l'IL1A tel que défini par le numéro de référence GenBank X03833, et
   (ii) un génotype homozygote G en position 6282 de l'exon 5 du gène de l'IL1A tel que défini par le numéro de référence GenBank X03833.

2. Procédé de prévision d'une hépatotoxicité induite par un médicament à la N-benzoyl-staurosporine chez un sujet, comprenant l'étape consistant à :

   déterminer *in vitro* le génotype d'un sujet au niveau des loci génétiques d'IL1A,

   (i) en position 549 du promoteur du gène de l'IL1A tel que défini par le numéro de référence GenBank X03833, et
   (ii) en position 6282 de l'exon 5 du gène de l'Il1A tel que défini par le numéro de référence GenBank X03833,

   où un génotype homozygote C en position 549 du promoteur du gène de l'IL1A tel que défini par le numéro de référence GenBank X03833 et un génotype homozygote G en position 6282 de l'exon 5 du gène de l'IL1A tel que défini par le numéro de référence GenBank X03833 sont présymptomatiques d'un risque d'hépatotoxicité induite par un médicament à la N-benzoyl-staurosporine.

**FIG. 1: AST/ALT Maximum Levels v *IL1A* (PG locus ID 279)**

FIG. 2: AST/ALT Maximum Levels v *IL1A* (PG locus ID 302)

## REFERENCES CITED IN THE DESCRIPTION

**Patent documents cited in the description**

- US 6214819 B
- US 20030119812 A
- US 20030125343 A
- US 20030153551 A
- US 5093330 A
- EP 730663 A
- EP 717113 A
- US 9702102 W
- US 5744460 A
- US 5827846 A
- US 6018042 A
- US 6153599 A
- US 6214819 A
- US 6300076 B1
- US 6297018 B1
- US 6300063 B1
- US 5846717 A
- US 6001567 A
- WO 9820020 A
- WO 9820019 A
- US 4965188 A
- WO 9001069 A

- US 5130238 A
- EP 329822 A
- US 5169766 A
- WO 8906700 A
- WO 9511995 A
- WO 9215712 A
- US 5679524 A
- WO 9102087 A
- WO 9009455 A
- WO 9517676 A
- US 5302509 A
- US 5945283 A
- US 5605798 A
- WO 9322456 A
- WO 8910414 A
- US 5866404 A
- US 5610053 A
- US 4843155 A
- US 4683202 A, Mullis
- US 1987 A
- US 5854033 A
- US 1988 A

**Non-patent literature cited in the description**

- **Way KJ et al.** *Diabetic Medicine,* 2001, vol. 18, 945-59
- **Koya D ; King GL.** *Diabetes,* 1998, vol. 47, 859-866
- **Ishii H et al.** *J Mol Med,* 1998, vol. 76, 21-31
- **Way KJ et al.** *Trends Pharmacol Sci,* 2000, vol. 21, 181-7
- **Dunlop ME ; Larkins RG.** *Biochem Biophys Res Commun,* 1985, vol. 132, 467-73
- **Aiello LP et al.** *Diabetes,* 1997, vol. 46, 1473-80
- PCR Technology: Principles and Applications for DNA Amplification. Freeman Press, 1992
- PCR Protocols: A Guide to Methods and Application. Academic Press, 1990
- **Kan ; Dozy.** *Lancet,* 1978, vol. II, 910-912
- **Wallace et al.** *Nucl. Acids Res.,* 1978, vol. 6, 3543-3557
- **Saiki et al.** *Proc. Natl. Acad. Sci. USA,* 1969, vol. 86, 6230-6234
- **Maskos ; Southern.** *Nucl. Acids Res.,* 1993, vol. 21, 2269-2270

- **Newton et al.** *Nucl. Acids Res.,* 1989, vol. 17, 2503-2516
- **Faham ; Cox.** *Genome Res.,* 1995, vol. 5, 474-482
- **Wagner et al.** *Nucl. Acids Res.,* 1995, vol. 23, 3944-3948
- **Fisher ; Lerman.** *Proc. Natl. Acad. Sci. U.S.A.,* 1983, vol. 80, 1579-1583
- **Orita et al.** *Genomics,* 1983, vol. 5, 874-879
- **Myers et al.** *Science,* 1985, vol. 230, 1242
- **Cotton et al.** *Proc. Natl. Acad. Sci. U.S.A.,* 1988, vol. 8Z, 4397-4401
- **Youil et al.** *Proc. Natl. Acad. Sci. U.S.A.,* 1995, vol. 92, 87-91
- **Syvanen et al.** *Genomics,* 1990, vol. 8, 684-692
- **Nikiforov et al.** *Nucl. Acids Res.,* 1994, vol. 22, 4167-4175
- **Landegren et al.** *Science,* vol. 241, 1077
- **Barrany.** *Proc. Natl. Acad. Sci. U.S.A.,* 1991, vol. 88, 189-193
- **Abravaya et al.** *Nucl. Acids Res.,* 1995, vol. 23, 675-682

- **Orum et al.** *Nucl. Acids Res.,* 1993, vol. 21, 5332-5356
- **Thiede et al.** *Nucl. Acids Res.,* 1996, vol. 24, 983-984
- **Sambrook J et al.** Molecular Cloning: A Laboratory Manual. Cold Spring Harbor Press, 2000
- **Wang et al.** *Science,* 1998, vol. 280, 1077-1082
- **Nowotny P.** *Current Opinions in Neurobiology,* 2001, vol. 11, 637-641
- **Shi MM.** *Clin Chem,* 2001, vol. 47, 164-172
- **Cox et al.** *Hum Mutat,* 2001, vol. 17, 141-150
- **Chan X et al.** *Genome Res,* 1999, vol. 9, 492-499
- **Ahmadiian A et al.** *Anal Biochem,* 2000, vol. 280, 103-10
- **Ryan D et al.** *Molecular Diagnosis,* 1999, vol. 4 (2), 135-144
- **Lyamichev V et al.** *Nature Biotechnology,* 1999, vol. 17, 292-296
- **Stevens JC.** *Mol Diag,* 1999, vol. 4, 309-317
- **Barany et al.** *Proc Natl Acad Sci USA,* 1991, vol. 88, 189-193
- **Landegren et al.** *Science,* 1988, vol. 241, 1077-1080
- **Walker et al.** *Proc Natl Acad Sci USA,* 1992, vol. 89, 392-396
- **Winter et al.** *Proc Natl Acad Sci USA,* 1985, vol. 82, 7575
- **Meyers et al.** *Science,* 1985, vol. 230, 1242
- **Modrich P.** *Ann Rev Genet,* 1991, vol. 25, 229-253
- **Orita et al.** *Genomics,* 1989, vol. 5, 874-879
- **Humphries et al.** Molecular Diagnosis of Genetic Diseases. 1996, 321-340
- **Wartell et al.** *Nucl Acids Res,* 1990, vol. 18, 2699-2706
- **Sheffield et al.** *Proc Natl Acad Sci USA,* 1989, vol. 86, 232-236
- **Ruafio et al.** *Nucl Acids Res,* 1989, vol. 17, 8392
- **Ruafio et al.** *Nucl Acids Res,* 1991, vol. 19, 6877-6882
- **Turki et al.** *J Clin Invest,* 1995, vol. 95, 1635-1641
- **D.L. Hartl et al.** Principles of Population Genomics. Sinauer Associates, 1997
- **Michalotos-Beloin et al.** *Nucl Acids Res,* 1996, vol. 24, 4841-4843
- **Bailey NTJ.** Statistical Methods in Biology. Cambridge Univ. Press, 1997
- **Waterman MS.** Introduction to Computational Biology. CRC Press, 2000
- Bioinformatics. John Wiley & Sons, Inc, 2001
- **L.D. Fisher ; G. vanBelle.** Biostatistics: A Methodology for the Health Sciences. Wiley-Interscience, 1993
- Genetic Algorithms and Their Uses in Chemistry. **R. Judson.** Reviews in Computational Chemistry. VCH Publishers, 1997, vol. 10, 1-73
- **Press et al.** Numerical Recipes in C: The Art of Scientific Computing. Cambridge University Press, 1992
- **E. Rich ; K. Knight.** Artificial Intelligence. McGraw-Hill, 1991
- In: Recombinant DNA. W.H. Freeman and Company, 254-272
- Current Protocols in Molecular Biology. 1997, vol. I-III
- **Sambrook et al.** Molecular Cloning: A Laboratory Manual. Cold Spring Harbor Laboratory Press, 1989
- DNA Cloning: A Practical Approach. 1985, vol. I,II
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- A Practical Guide to Molecular Cloning. **Perbal.** Methods in Enzymol. Academic Press, Inc, 1984
- Gene Transfer Vectors for Mammalian Cells. Cold Spring Harbor Laboratory, 1987
- Methods in Enzymology. vol. 154,155
- **Tom Strachan ; Andrew, Read.** Human Molecular Genetics. John Wiley and Sons, Inc. Publication, 1999
- Curr. Prot. Mol. Biol. John Wiley & Sons, 1987
- **Guatelli et al.** *Proc. Natl. Acad. Sci. USA,* 1990, vol. 87, 1874-1878
- **Kwoh et al.** *Proc. Natl. Acad. Sci. USA,* 1989, vol. 86, 1173-1177
- **Lizardi et al.** *Biol. Technology,* 1988, vol. 6, 1197
- **Lyamichev V et al.** *Nat Biotechnol,* 1999, vol. 17, 292-6
- **Ryan D et al.** *Mol Diagn,* 1999, vol. 4, 135-44 9
- **Hochepied T et al.** *Cytokine Growth Factor Rev,* 2003, vol. 14, 25-34
- **Israili ZH ; Dayton PG.** *Drug Met Rev,* 2001, vol. 33, 161-235
- **Webb GC et al.** *Cytogenet Cell Genet,* 1998, vol. 47, 18-21
- **Yuasa I et al.** *Hum Genet,* 1997, vol. 99, 393-8
- **Spurdle AB et al.** *Pharmacogenetics,* July 2002, vol. 12, 355-66
- **Goodwin B et al.** *Annu Rev Pharmacol Toxicol,* 2002, vol. 42, 1-23
- **Jarvelainen HA et al.** *Hepatology,* 2001, vol. 33, 1148-53
- **Ramadori G ; Christ B.** *Semin Liver Dis,* 1999, vol. 19, 141-55

- **Jouvenne P et al.** *Eur Cytokine Netw,* 1999, vol. 10, 33-6
- **Jaeschke H et al.** *Toxicol Sci,* 2002, vol. 65, 166-76
- **Paul WE.** Fundamental Immunology. Lippingcott-Raven Publishers, 1999

- **Nagakawa J et al.** *Immunopharmacol Immunotoxicol,* 1991, vol. 13, 485-98
- **Mandrup-Poulsen T et al.** *Cytokine,* 1993, vol. 5, 185-81
- **Vassiliadis S et al.** *Mediators Inflamm,* 1999, vol. 8, 85-91

SEQUENCE LISTING

<110> Novartis AG
McCullough, Karen
Wolfgang, Curt

<120> USE OF GENETIC POLYMORPHISMS TO PREDICT DRUG INDUCED HEPATOTOXICITY

<130> DV/4-33390A

<150> 60/508,972
<151> 2003-10-06

<160> 18

<170> FastSEQ for windows version 4.0

<210> 1
<211> 20
<212> DNA
<213> Homo sapiens

<220>
<221> variation
<222> (1)...(20)
<223> PG locus ID 279; interleukin 1, alpha gene (X03833) positions 541 - 560; "C" variant at position 549
<221> variation
<222> (9)...(0)
<223> C


<400> 1
aggcaacacc attgaaggct        20


<210> 2
<211> 20
<212> DNA
<213> Homo sapiens


<220>
<221> variation
<222> (1)...(20)
<223> PG locus ID 279; interleukin 1, alpha gene (X03833) positions 541 - 560; "T" variant at position 549
<221> variation
<222> (9)...(0)
<223> T


<400> 2
aggcaacatc attgaaggct        20


<210> 3
<211> 20
<212> DNA
<213> Homo sapiens


<220>
<221> variation
<222> (1)...(20)
<223> PG locus ID 302; interleukin 1, alpha gene (X03833) positions 6270 - 6290; "G" variant at position 6282
<221> variation
<222> (12)...(0)
<223> G


<400> 3
agcctaggtc agcacctttt        20


<210> 4
<211> 20
<212> DNA
<213> Homo sapiens


<220>
<221> variation
<222> (1)...(20)
<223> PG locus ID 302; interleukin 1, alpha gene (X03833) positions 6270 - 6290; "T" variant at position 6282
<221> variation
<222> (12) ... (0)
<223> T


<400> 4
agcctaggtc atcacctttt        20

<210> 5
<211> 19
<212> DNA
<213> Homo sapiens

<220>
<221> primer_bind
<222> (1)...(19)
<223> PCR primer 2D6L1F1

<400> 5
ctgggctggg agcagcctc          19

<210> 6
<211> 23
<212> DNA
<213> Homo sapiens

<220>
<221> primer_bind
<222> (1)...(23)

<223> PCR primer 2D6L1R1

<400> 6
cactcgctgg cctgtttcat gtc          23

<210> 7
<211> 22
<212> DNA
<213> Homo sapiens

<220>
<221> primer_bind
<222> (1)...(22)
<223> PCR primer 2D6L2F

<400> 7
ctggaatccg gtgtcgaagt gg          22

<210> 8
<211> 20
<212> DNA
<213> Homo sapiens

<220>
<221> primer_bind
<222> (1)...(20)
<223> PCR primer 2D6L2R2

<400> 8
ctcggcccct gcactgtttc          20

<210> 9
<211> 22
<212> DNA
<213> Homo sapiens

```
<220>
<221> primer_bind
<222> (1)...(22)
<223> PCR primer 2D6L3F

<400> 9
gaggcaagaa ggagtgtcag gg          22


<210> 10
<211> 23
<212> DNA
<213> Homo sapiens

<220>
<221> primer_bind
<222> (1)...(23)
<223> PCR primer 2D6L3R5B

<400> 10
agtcctgtgg tgaggtgacg agg          23


<210> 11
<211> 19
<212> DNA
<213> Homo sapiens

<220>
<221> primer_bind
<222> (1)...(19)
<223> PCR primer CYP3A4Exon10F

<400> 11
tggatggccc acattctcg          19


<210> 12
<211> 20
<212> DNA
<213> Homo sapiens

<220>
<221> primer_bind
<222> (1)...(20)
<223> PCR primer CYP3A4Exon10R

<400> 12
cttcctacat agagtcagtg          20


<210> 13
<211> 21
<212> DNA
<213> Homo sapiens

<220>
<221> primer_bind
<222> (1)...(21)
<223> PCR primer PXR 252 F

<400> 13
```

ggacacagag tctgttcctg g     21

<210> 14
<211> 22
<212> DNA
<213> Homo sapiens

<220>
<221> primer_bind
<222> (1)...(22)
<223> PCR primer PXR 252 R

<400> 14
gaagatgaag gattcctctg gg     22

<210> 15
<211> 23
<212> DNA
<213> Homo sapiens

<220>
<221> primer_bind
<222> (1)...(23)
<223> PXR 11156 F

<400> 15
gacaaggcta cgctgacaat cag     23

<210> 16
<211> 24
<212> DNA
<213> Homo sapiens

<220>
<221> primer_bind <222> (1)...(24)
<223> PXR 11156 R

<400> 16
gcttgcgtat gtttctattt ccac     24

<210> 17
<211> 21
<212> DNA
<213> Homo sapiens

<220>
<221> primer_bind
<222> (1)...(21)
<223> PXR 24113 F

<400> 17
cggagcaaag aacttaccac c     21

<210> 18
<211> 21
<212> DNA
<213> Homo sapiens

<220>
<221> primer_bind
<222> (1) ... (21)
<223> PXR 24113 R

<400> 18
tgcaggacca gagagcatca g        21